(19) Europäisches Patentamt
European Patent Office
Office européen des brevets

(11) Veröffentlichungsnummer: **0 094 052**
**A2**

# (12) EUROPÄISCHE PATENTANMELDUNG

(21) Anmeldenummer: **83104456.5**

(22) Anmeldetag: **06.05.83**

(51) Int. Cl.³: **C 07 D 405/06**, C 07 D 405/12 // A61K31/445, C07D317/20, C07D317/18

(30) Priorität: **12.05.82 DE 3217770**
**22.10.82 DE 3239170**
**22.10.82 DE 3239169**

(43) Veröffentlichungstag der Anmeldung: **16.11.83**
**Patentblatt 83/46**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Winkelmann, Erhardt, Dr., Brunhildenweg 5, D-6233 Kelkheim (Taunus) (DE)**
Erfinder: **Raether, Wolfgang, Dr., Falkensteinstrasse 6, D-6072 Dreieich (DE)**
Erfinder: **Dittmar, Walter, Dr., Uhlandstrasse 10, D-6238 Hofheim am Taunus (DE)**

(54) **1-(1,3-Dioxolan-2-yl-methyl)-1H-imidazole und -1H-1,2,4-triazole und deren Salze, Verfahren zu ihrer Herstellung, sie enthaltende Mittel und ihre Verwendung.**

(57) Gegenstand der Erfindung sind neue 1-(1,3-Dioxolan-2-yl-methyl)-1H-imidazole und -1H-1,2,4-triazole der Formel I

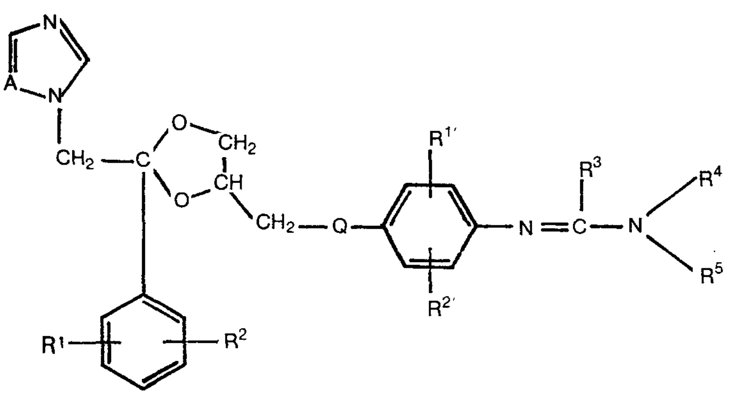

(I)

und ihre Salze mit physiologisch verträglichen Säuren, sowie Verfahren zur Herstellung dieser Verbindungen. Die neuen Verbindungen wirken gegen Infektionen durch Pilze wie Hautpilze, Schimmelpilze oder Hefearten; außerdem wirken sie gegen Bakterien und Protozoen.

EP 0 094 052 A2

HOECHST AKTIENGESELLSCHAFT HOE 82/F 094 K Dr.KM/cr

# 1-(1,3-Dioxolan-2-yl-methyl)-1H-imidazole und -1H-1,2,4-triazole und deren Salze, Verfahren zu ihrer Herstellung, sie enthaltende Mittel und ihre Verwendung

In DE-OS 2.804.096 sind 1-(1,3-Dioxolan-2-yl-methyl)-1H-imidazole beschrieben, die zur Bekämpfung von Pilzen und Bakterien geeignet sind. In J. Med. Chem. 1979, Vol. 22, No. 8, 1003 wird die Darstellung und antimykotische Wirkung von Cis-1-acetyl-4-/4-/2-(2,4-dichlorphenyl)-2-(1H-imidazol-1-yl-methyl)-1,3-dioxolan-4-yl-methoxy/-phenyl/-piperazin = Ketoconazol beschrieben.

Gegenstand der Erfindung sind 1-(1,3-Dioxolan-2-yl-methyl)-1H-imidazole und -1H-1,2,4-triazole der Formel I

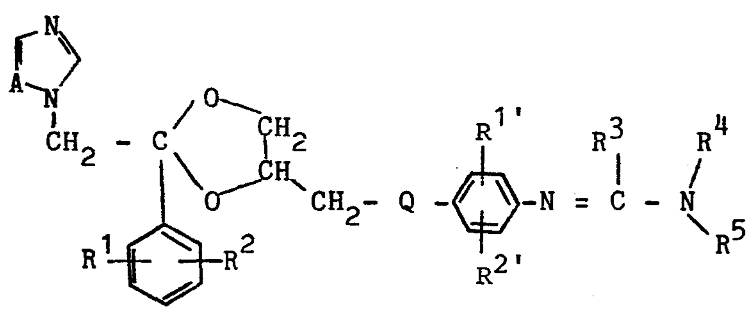

(I)

und ihre Salze mit physiologisch verträglichen Säuren, worin A Stickstoff oder eine Methingruppe, Q Sauerstoff, Schwefel, eine SO-Gruppe (Sulfoxid) oder eine $SO_2$-Gruppe (Sulfon),
$R^1$ und $R^2$, $R^{1'}$ und $R^{2'}$ gleich oder verschieden sein können und Wasserstoff, Halogen, insbesondere Fluor und Chlor, $C_1$-$C_4$-Alkyl, insbesondere Methyl und Äthyl, $C_1$-$C_4$-Alkoxy, insbesondere Methoxy und Äthoxy, oder Trifluormethyl,
$R^3$, $R^4$, $R^5$ gleich oder verschieden sein können und Wasserstoff, geradkettiges oder verzweigtes Alkyl mit 1 bis

4 Kohlenstoffatomen, wie Methyl, Äthyl, Propyl, Isopropyl, Butyl, Isobutyl bedeuten, oder worin $R^3$ und $R^4$ als Alkylenkette mit drei bis fünf Kohlenstoffatomen zusammen mit dem Stickstoff- und Kohlenstoffatom der Amidinogruppe Bestandteil eines Pyrrolidin-, Piperidin- oder Hexamethylenimin-Ringes sind, oder worin $R^4$ und $R^5$ zusammen eine Alkylenkette mit vier bis sechs Kohlenstoffatomen, die mit dem Stickstoffatom einen fünf- bis siebengliedrigen heterocyclischen Ring bilden, der ein weiteres Heteroatom aus der Gruppe Stickstoff, Sauerstoff oder Schwefel enthalten kann und seinerseits durch Alkyl mit ein bis drei Kohlenstoffatomen, wie Methyl, Äthyl, Propyl oder Aralkyl, wie Benzyl, oder Aryl wie Phenyl, Tolyl, Methoxyphenyl, Chlorphenyl, Trifluormethylphenyl, substituiert sein kann, insbesondere Pyrrolidin, 2-Methyl-pyrrolidin, 2,5-Dimethylpyrrolidin, Piperidin, 2-Methylpiperidin, 4-Methyl-piperidin, 2,6-Dimethylpiperidin, Morpholin, 2-Methyl-morpholin, 2,6-Dimethylmorpholin, Thiomorpholin, Piperazin, N-Methyl-piperazin, N-Äthyl-piperazin, N-Benzyl-piperazin, N-Phenyl-piperazin, N-4-Tolyl-piperazin, N-4-Methoxyphenyl-piperazin, N-4-Chlorphenyl-piperazin oder N-3-Trifluormethylphenyl-piperazin, bedeuten.

Bevorzugt sind Verbindungen der Formel I, in welcher sich $R^1$ und $R^2$ in 2,4-Position befinden und Chlor, $R^{1'}$ und $R^{2'}$ Wasserstoff bedeuten, $R^3$ Wasserstoff oder Methyl, die substituierte Aminogruppe $-N-R^4-(R^5)$ Dimethylamino, Diäthylamino, Pyrrolidin, Piperidin oder Morpholin, und A eine Methingruppe (Imidazolring) und Q Sauerstoff oder Schwefel bedeuten.

Gegenstand der Erfindung ist auch ein Verfahren zur Herstellung von 1-(1,3-Dioxolan-2-yl-methyl)1H-imidazolen und -1H-1,2,4-triazolen der Formel I und ihrer Salze, das dadurch gekennzeichnet ist, daß man

A) ein 4-[2-(subst.-Phenyl)-2-(1H-azol-1-yl-methyl)-1,3-dioxolan-4-yl-methoxy]-phenylamin der Formel II

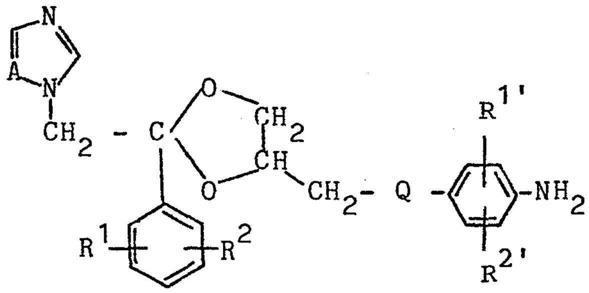

(II)

worin A,Q, $R^1$, $R^2$, $R^{1'}$ und $R^{2'}$ die zu Formel I angegebenen Bedeutungen haben,

a) mit einem Carbonsäureamid, Carbonsäurethioamid, Lactam oder Thiolactam der Formel III

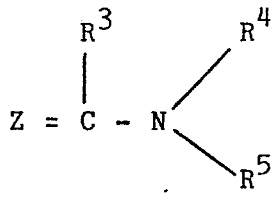

(III)

worin Z Sauerstoff oder Schwefel bedeutet und $R^3$, $R^4$ und $R^5$ die zu Formel I angegebenen Bedeutungen haben, in Gegenwart eines Kondensationsmittels umsetzt, oder

b) mit einem Acetal eines Carbonsäureamids oder eines Lactams der Formel IV

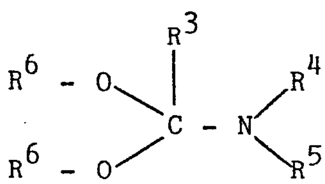

(IV)

worin $R^3$, $R^4$, $R^5$ die zu Formel I angegebenen Bedeutungen haben und $R^6$ Methyl oder Äthyl bedeutet, umsetzt, oder

c) mit einem Imidoäther oder Imidothioäther der Formel V

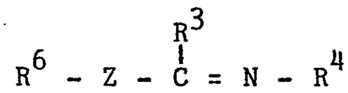

(V)

worin Z, $R^3$, $R^4$, $R^6$ die vorstehend angegebenen Bedeutungen haben, umgesetzt, oder

d) mit einem Orthoester der Formel VI

$$R^3 - C(OR^6)_3 \qquad (VI)$$

worin $R^3$ und $R^6$ die angegebenen Bedeutungen haben, in Gegenwart eines Kondensationsmittels zu einem Imidoäther der Formel VII umsetzt

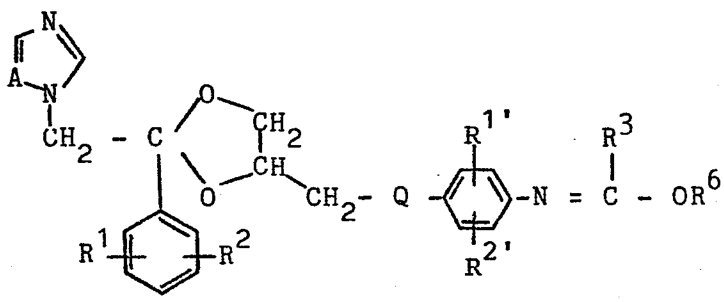

(VII)

worin A,Q, $R^1$, $R^2$, $R^{1'}$, $R^{2'}$, $R^3$ und $R^6$ die angegebenen Bedeutungen haben, und anschließend die so erhaltene Verbindung VII mit einem Amin der Formel VIII

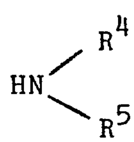

(VIII)

worin $R^4$ und $R^5$ die angegebenen Bedeutungen haben, umsetzt, oder

e) mit Trichloracetaldehyd zu einer Trichloräthyliden-Verbindung der Formel IX

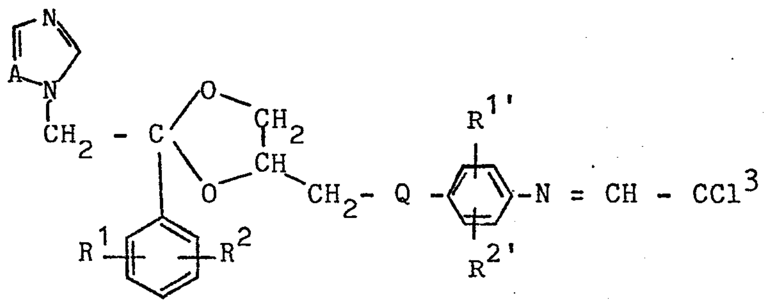

(IX)

worin A,Q, $R^1$, $R^2$, $R^{1'}$, $R^{2'}$ die angegebenen Bedeutungen haben, umsetzt und anschließend mit einem Amin der Formel VIII umsetzt, oder

f) mit einem entsprechenden Säurechlorid oder Säureanhydrid zu einem Amid der Formel X

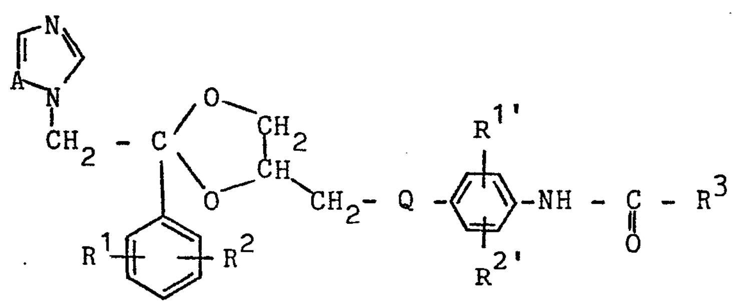

(X)

worin A,Q, $R^1$, $R^2$, $R^{1'}$, $R^{2'}$,und $R^3$ die angegebenen Bedeutungen haben umsetzt,anschließend die Verbindung X mit einem Phosphorchlorid zu einem Imidchlorid der Formel XI

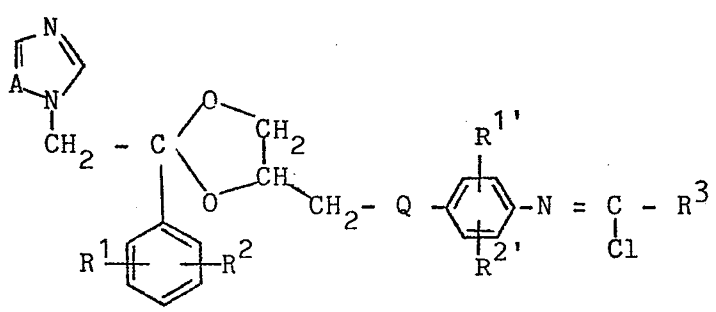

(XI)

worin A,Q, $R^1$, $R^2$, $R^{1'}$, $R^{2'}$ und $R^3$ die angegebenen Bedeutungen haben, umsetzt und dieses mit einem Amin der Formel VIII umsetzt, oder

g) mit einem ω-Halogencarbonsäurechlorid oder -anhydrid zu einem Amid der Formel XII

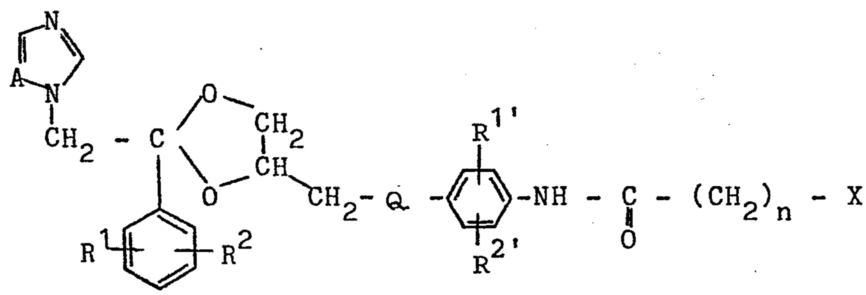
(XII)

worin A,Q, $R^1$, $R^2$, $R^{1'}$ und $R^{2'}$ die angegebenen Bedeutungen haben und n die Zahl drei bis fünf und X Halogen wie Fluor, Chlor, Brom, Jod oder Acyloxy, wie Acetoxy-,Benzoyloxy, Nitrobenzoyloxy-, Alkylsulfonyloxy, wie Methansulfonyloxy-, oder Arylsulfonyloxy, wie Benzolsulfonyloxy-, Toluolsulfonyloxy, Nitrobenzolsulfonyloxybedeutet, umsetzt und anschließend dieses mit einem Phosphorchlorid zu einem Imidchlorid der Formel XIII

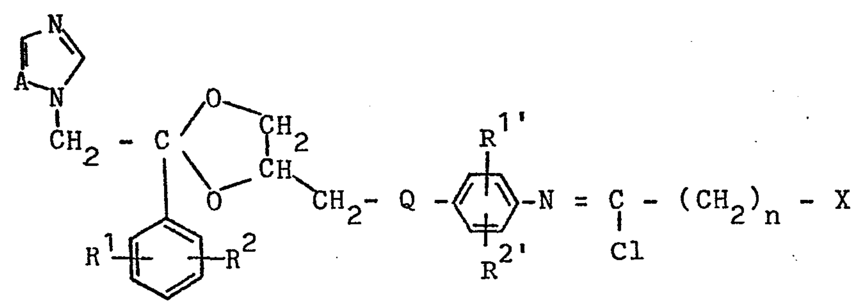
(XIII)

worin A, Q, $R^1$, $R^2$, $R^{1'}$, $R^{2'}$, n und X die angegebenen Bedeutungen haben, umsetzt und anschließend dieses mit einem Amin der Formel XIV

$$H_2N - R^4 \qquad (XIV)$$

worin $R^4$ die angegebene Bedeutung hat, umsetzt, oder daß man

B)a) ein 2-(subst.-Phenyl)-2-(1H-azol-1-yl-methyl)-1,3-dioxolan der Formel XV

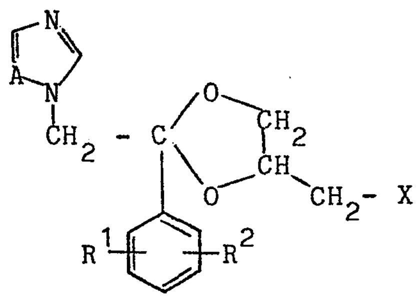

(XV)

worin A, $R^1$, $R^2$ und X die angegebenen Bedeutungen haben, mit einem 4-Amidinophenol oder mit seinem Alkalimetall- oder Ammoniumsalz der Formel XVI

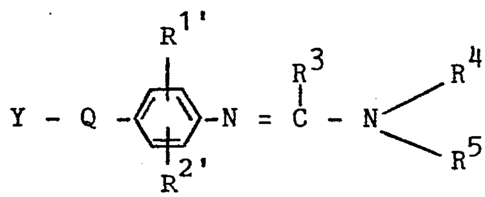

(XVI)

worin Y Wasserstoff, ein Alkalimetall, insbesondere Natrium oder Kalium, oder Ammonium bedeutet und Q,$R^{1'}$, $R^{2'}$, $R^3$, $R^4$, $R^5$, die angegebenen Bedeutungen haben, umsetzt, oder daß man

b) ein 2-(subst.-Phenyl)-2-brommethyl-1,3-dioxolan der Formel XVII

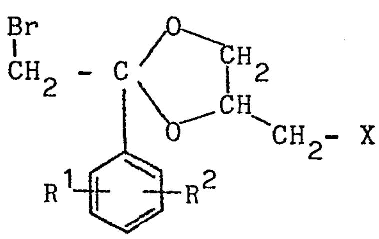

(XVII)

worin $R^1$, $R^2$ und X die angegebenen Bedeutungen haben, mit einem 4-Amidinophenol der Formel XVI zu einer Verbindung der Formel XVIII

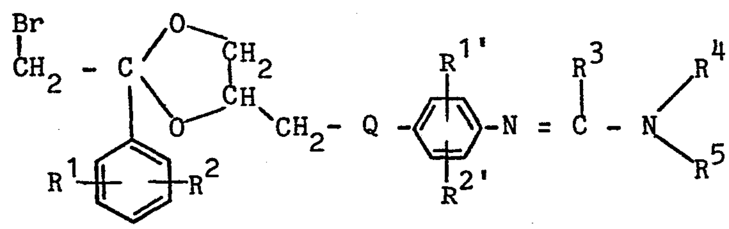

(XVIII)

worin Q, $R^1$, $R^2$, $R^{1'}$, $R^{2'}$, $R^3$, $R^4$ und $R^5$ die angegebenen Bedeutungen haben, umsetzt und diese anschließend mit einem Azol der Formel XIX

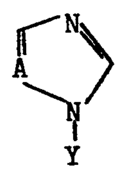

(XIX)

worin A und Y die angegebene Bedeutung haben, umsetzt, oder daß man

C) ein $\omega$-Bromacetophenon der Formel XX

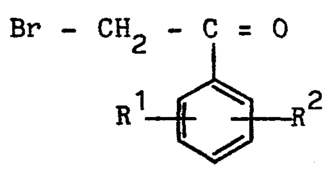

(XX)

worin $R^1$ und $R^2$ die angegebenen Bedeutungen haben, mit $\alpha$-Glycerin-4-nitrophenyläther der Formel XXI

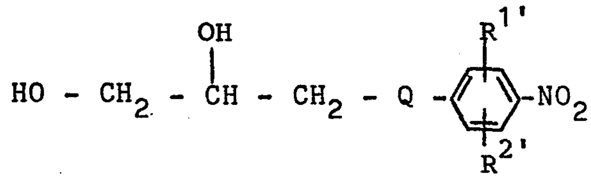

(XXI)

zu einem Ketal der Formel XXII

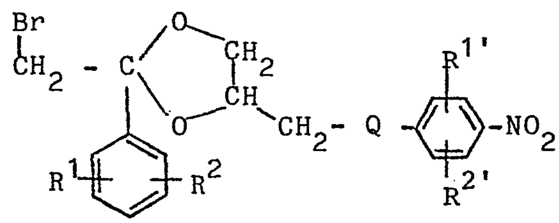

(XXII)

worin Q, $R^1$, $R^2$, $R^{1'}$ und $R^{2'}$ die angegebenen Bedeutungen haben, umsetzt, und dieses anschließend zu einer Aminoverbindung der Formel XXIV

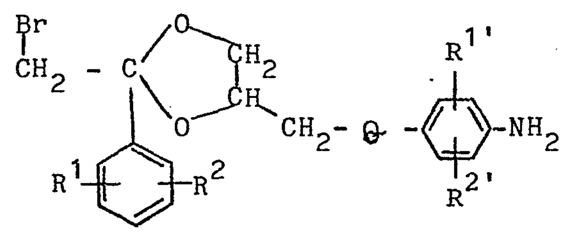

(XXIII)

worin Q, $R^1$, $R^2$, $R^{1'}$ und $R^{2'}$ die angegebenen Bedeutungen haben, reduziert und danach mit einer Verbindung der Formel III in Gegenwart eines Kondensationsmittels in eine Verbindung der Formel XVIII umwandelt und diese schließlich mit einem Azol der Formel XIX umsetzt, und gegebenenfalls die so erhaltene Verbindung der Formel I durch Zugabe einer physiologisch verträglichen Säure in das Salz überführt.

Zur Herstellung der Ausgangsstoffe der Formel II wird auch der vorstehend beschriebene Syntheseweg zu den Verbindungen der Formel XXII beschritten. Anschließend wird ein Ketal der Formel XXII mit einem Azol der Formel XIX zu einer Verbindung der Formel XXIV

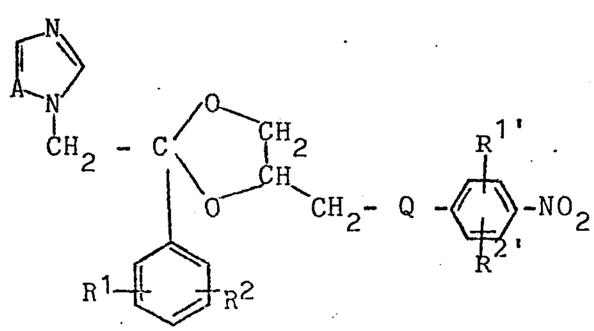

(XXIV)

worin A,Q, $R^1$, $R^2$, $R^{1'}$ und $R^{2'}$ die angegebenen Bedeutungen haben, umgesetzt und danach anschließend zu einer Aminoverbindung der Formel II reduziert.Die Ausgangsstoffe der Formel II sind nach DE-OS 28 04 096 bzw. Europ. Pat. 52.905 bekannt.
Als Ausgangsstoffe der Formel III (Carbonsäureamide und Thioamide) kommen beispielsweise in Frage Formamid, Thioformamid, N-Methyl-, N-Äthyl-, N-Propyl-, N-Isopropyl-, N-Butyl-, N-Isobutyl-, N,N-Dimethyl-, N,N-Diäthyl, N,N-Dipropyl, N,N-Diisopropyl, N,N-Dibutyl-, N,N-Diisobutylformamid, -thioformamid, -acetamid, -thioacetamid, propionamid, -thiopropionamid, -butyramid, -thiobutyramid, -valeramid, -thiovaleramid, ferner N-Formyl-, N-Acetyl-, N-Propionyl-, N-Butyryl-, N-Valerylpyrrolidin, -piperidin, -morpholin, -thiomorpholin, Piperazin, N-Methyl-piperazin, N-Äthylpiperazin, N-Benzyl-piperazin, N-Phenyl-piperazin, N-4-Tolyl-piperazin, N-4-Methoxyphenyl-piperain, N-4-Chlorphenyl-piperazin oder N-3-Trifluormethylphenyl-piperazin.

Als weitere Ausgangsstoffe der Formel III (Lactame und Thiolactame) kommen beispielsweise in Frage Butyrolactam (Pyrrolidon-2), Valerolactam (Piperidon-2), Caprolactam (2-Oxohexamethylenimin), Butyro-, Valero-, Caprothiolactam, N-Methyl-, N-Äthyl, N-Propyl-, N-Butyl-butyro-, -Valero-, -capro-lactam, -butyro-, -valero-, -capro-thiolactam.

Als Ausgangsstoffe der Formel IV kommen beispielsweise in Frage Formamid, N-Methyl, N-Äthyl-, N-Propyl, N-Isopropyl, N-Butyl, N-Isobutyl, N,N-Dimethyl-, N,N-Diäthyl-, N,N-Dipropyl-, N,N-Diisopropyl-, N,N-Dibutyl-, N,N-Diisobutylformamid-, -acetamid-, -propionamid-, -butyramid, -valeramid-, dimethyl-, diäthylacetal, ferner N-Formyl-, N-Acetyl-, N-Propionyl-, N-Butyryl-, N-Valeryl-pyrrolidin-, -piperidin-, morpholin-, -thiomorpholin-, Piperazin, N-Methyl-piperazin, N-Äthyl-piperazin, N-Benzyl-piperazin, N-Phenyl-piperazin, N-4-Tolyl-piperazin, N-4-Methoxyphenyl-

piperazin, N-4-Chlorphenyl-piperazin, N-3-Trifluormethylphenyl-piperazin,-dimethyl-, -diäthyl-acetal,ferner Butyrolactam-, (Pyrrolidon-2)-, Valerolactam-, (Piperidon-2)-, Caprolactam-, (2-Oxohexamethylenimin)-, Butyro-, Valero-, Caprothiolactam-, N-Methyl-, N-Äthyl-, N-Propyl-, N-Butyl--butyro-, -valero-, -capro-lactam, -butyro-, -valero-, -capro-thiolactam-, -dimethyl- oder -diäthyl-acetal.

Als Ausgangsstoffe der Formel V kommen beispielsweise in Frage N-Methyl-, N-Äthyl-, N-Propyl-, N-Isopropyl-, N-Butyl-, N-Isobutyl-, -formamid-, -acetamid-, -propionamid-, -butyramid-, -valeramid-, -methyl-, äthyl-imidoäther oder-imidothioäther, Butyrolactam, (= Pyrrolidon-2), Valerolactam-, (= Piperidon-2), Caprolactam-, (= 2-Oxohexamethylenimin), -methyl-, -äthyl-imidoäther oder -imidothioäther.

Als Ausgangsstoffe der Formel VI kommen beispielsweise in Frage Ameisensäure-, Essigsäure-, Propionsäure-, Buttersäure-, Valeriansäure-, -trimethyl- oder -triäthyl-orthoester.

Die Ausgangsstoffe der Formel III, IV, V, VI, VIII, XIV sowie ihre Herstellung sind bekannt.

Die Amide der Formel III können auf bekannte Weise durch Formylierung oder Acylierung der entsprechenden Amine, die Thioamide der Formel III aus entsprechenden Amiden durch Schwefelung, z. B. mittels Phosphorpentasulfid, dargestellt werden.

Die Acetale der Formel IV können auf bekannt Weise durch Umsetzung der entsprechenden Amide der Formel III mit Bortrifluoridätherat (Methode nach Meerwein) dargestellt werden.

Die Imidoäther der Formel V können auf bekannte Weise durch Alkylierung der entsprechenden Amide der Formel II mittels Dialkylsulfat dargestellt werden.
Die Imidothioäther der Formel V können auf bekannte Weise durch Umsetzung der entsprechenden Imidchloriden (aus Amiden III und Phosphorpentachlorid) mit Alkylmercaptanen dargestellt werden.

Als Imidoäther der Formel VII kommen beispielsweise in Frage Ameisensäure-, Essigsäure-, Propionsäure-, Buttersäure-, Valeriansäure-, -methyl-, -äthyl-imidoäther.
Die Herstellung erfolgt auf bekannte Weise durch Umsetzung von Anilinen der Formel II mit entsprechenden Orthoestern in Gegenwart eines Kondensationsmittels.

Die Trichloräthyliden-Verbindungen der Formel IX werden auf bekannte Weise durch Umsetzung von Anilinen der Formel II mit Chloral (Trichloracetaldehyd) dargestellt.

Als Ausgangsstoffe der Formel XII kommen beispielsweise in Frage Chlor-, Brom-, Jod-acetamide, ω-Halogen-propionsäure-, -buttersäure-, -valeriansäure-, -capronsäure-amide.
Die Herstellung der Amide der Formel XII erfolgt durch Umsetzung von Anilinen der Formel II mit entsprechenden ω-Halogen-carbonsäurechloriden- oder -anhydriden.

Als Ausgangsstoffe der Formel XIII kommen beispielsweise in Frage ω-Halogen-essigsäure-, -propionsäure-, -buttersäure-, -valeriansäure- oder -capronsäure-imidchloride.
Die Herstellung der Imidchloride erfolgt durch Umsetzung der Amide der Formel XII mit Phosphorchloriden, vorzugsweise Phosphor-pentachlorid.

Als Ausgangsstoffe der Formel XV bzw. XVII kommen beispielsweise in Frage 4-Chlormethyl-, 4-Brommethyl-, 4-Benzoyloxymethyl-, 4-(4-Nitrobenzoyloxy)-methyl-, 4-Methansulfonyloxymethyl-, 4-(4-Toluolsulfonyloxy)-methyl- oder 4-(4-Nitrobenzolsulfonyloxy)-methyl-1,3-dioxolane.
Die Herstellung dieser reaktiven Dioxolane erfolgt aus den entsprechenden 4-Hydroxymethyl-Verbindungen druch Umsetzung mit z. B. Thionylchlorid, Thionylbromid, Benzoylchlorid, 4-Nitrobenzoylchlorid, Methansulfonylchlorid, 4-Toluolsulfonylchlorid, 4-Nitrobenzolsulfonylchlorid.

Als Ausgangsstoffe der Formel XVI bzw. XVIII kommen beispielsweise in Frage 4-Amino-, 4-Methylamino-, 4-Äthylamino-, 4-Propylamino-, 4-Isopropylamino-, -butylamino-, -isobutylamino-, -dimethylamino-, -diäthylamino-, 4-Di-n-propylamino-, 4-Di-isopropylamino-, 4-Di-n-butylamino-, 4-Di-isobutylamino-, 4-Pyrrolidino-, 4-Piperidino-, 4-Morpholino-, 4-Thiomorpholino-, Piperazino-, N-Methyl-piperazino-, N-Äthyl-piperazino-, N-Benzyl-piperazino-, N-Phenyl-piperazino-, N-4-Tolyl-, Piperazino-, N-4-Methoxyphenyl-piperazino-, N-4-Chlorphenyl-piperazino-, N-3-Trifluormethylphenyl-piperazino-, -methylenimino-, -1-äthylenimino-, -1-propylenimino-phenol, sowie 4-(pyrrolidon-2-imino)-, -(piperidon-2-imino)-, -(2-oxohexamethylenimino-2-imino)-, -(1-methyl-, 1-äthyl, 1-propyl-, 1-butyl-pyrrolidon-, -piperidon-, -2-oxohexamethylen-imino-2-imino)-phenol bzw.-phenoläther oder-thiophenol oder -thiophenolen bzw. -thiophenoläther.

Die Herstellung der 4-Amidino-phenole oder -thiophenole XVI erfolgt durch Umsetzung von 4-Benzyloxy-.oder thio-anilin mit entsprechenden Amiden der Formel lII in Gegenwart von Phosphoroxychlorid und anschließender Abspaltung der Benzylgruppe, z. B. mit Bromwasserstoffsäure in Eisessig.
Die Herstellung der 4-Amidino-phenoläther oder -thiophenoläther XVIII erfolgt durch Umsetzung der Ausgangsstoffe der Formel XVII mit 4-Amidinophenolen oder -thiophenolen der Formel XVI.

Die Ausgangsstoffe der Formel XX sind bekannt und werden nach üblichen Methoden durch Bromierung der entsprechenden Aecetophenone mit Brom in Eisessig gewonnen.

Die Ausgangsstoffe der Formel XXI mit Q gleich Sauerstoff sind bekannt (Bull. Soc. Chim. France (4), 13, Seite 526). Die anderen Verbindungen werden analog hergestellt.

Die Ketale der Formel XXII werden durch Ketalisierung von ω-Bromactophenonen XX mit α-Glycerin-4-nitrophenyläther oder -thioäther XXI unter wasserabspaltenden Bedingungen in Gegenwart eines Katalysators, wie 4-Toluolsulfonsäure erhalten.

Die Verbindungen der Formel XXIV werden durch Umsetzung der Bromketale XXII mit Azolen der Formel XIX in polaren Lösungsmitteln bei höheren Temperaturen erhalten.

Die Reduktion der Ausgangsstoffe XXIV (Nitroverbindungen) zu den Ausgangsstoffen II (Aminoverbindungen), sowie die der Ausgangsstoffe XXII (Nitroverbindungen) zu den Ausgangsstoffen XXIII (Aminoverbindungen) wird mit nascierendem Wasserstoff durchgeführt. Hierfür eignet sich besonders Zink-Staub in Gegenwart einer schwachen Säure, wie Essigsäure, oder einer Lewis-Säure wie Ammoniumchlorid oder Calciumchlorid. Die Reduktionsreaktion wird vorteilhaft in wäßrigem Alkohol bei erhöhter Temperatur durchgeführt. Eine Reduktion ist auch durch katalytisch angeregten Wasserstoff möglich. Als Katalysatoren eignen sich Nickel, Palladium oder Platin in feinverteiltem Zustand oder auf geeigneten Trägern.

Die Verbindungen der Formel XVIII erhält man auch durch Umsetzung der Aminoverbindungen der Formel XXIII mit Amiden der Formel III in Gegenwart eines Kondensationsmittels wie Phosphoroxichlorid.

Die Umsetzungen nach den Varianten A) und B) des Herstellungsverfahrens werden zweckmäßig in äquimolaren Mengen der jeweiligen Ausgangsstoffe durchgeführt. Bei flüchtigen Reaktionspartnern empfiehlt sich jedoch die Anwendung eines Überschusses. Die Umsetzungen werden vorteilhaft in einem Lösungs- oder Verteilungsmittel durchgeführt, jedoch sind bestimmte Reaktionen auch ohne Lösungs- oder Verteilungsmittel durchführbar, wie nachstehend ausgeführt wird.

Als Lösungs- oder Verteilungsmittel kommen beispielsweise in Frage:
Bei Verfahren A) a) aromatische, gegebenenfalls halogenierte Kohlenwasserstoffe, wie Benzol, Toluol, Xylol, Chlorbenzol, Dichlorbenzol, Trichlorbenzol, chlorierte, aliphatische Kohlenwasserstoffe, wie Methylenchlorid, Chloroform, aliphatische Äther, wie Di-isopropyläther, Äthylenglycoldimethyläther, -diäthyläther, Diäthylenglycol-dimethyläther, Tetrahydrofuran, Dioxan, Nitrile, wie Acetonitril, Propionitril oder Benzonitril.

Besonders vorteilhaft ist es, die zur Umsetzung verwendeten Carbonsäureamide oder Lactame der Formel III im Überschuß zu verwenden. Der Überschuß kann gegebenenfalls bei der Aufarbeitung des Reaktionsansatzes wiedergewonnen werden.

Bei Verfahren A) b) Alkohole, wie Methanol, Äthanol, Propanol, Butanol, Methoxyäthanol, Äthoxyäthanol, oder Pyridin, Picolin, Lutidin, Chinolin, Chinaldin, oder besonders vorteilhaft die zur Umsetzung verwendeten Acetale der Carbonsäureamide oder der Lactame der Formel IV im Überschuß.

Bei Verfahren A) c) Alkohole, wie Methanol, Äthanol, Propanol, Butanol, Methoxyäthanol, Äthoxyäthanol, Amide, wie Dimethylformamid, Dimethylacetamid, Tetramethylharnstoff, N-Methylpyrrolidon oder Eisessig. Besonders vorteilhaft ist die Verwendung der umzusetzenden Imidoäther der Formel V im Überschuß.

Bei Verfahren A) d) und A) e) werden vorteilhaft die umzusetzenden Orthoester der Formel VI und Amine der Formel VIII, sowie Trichloracetaldehyd und Amine der Formel VIII im Überschuß angewendet.

Bei Verfahren A) f), A) g) und B) Alkohole, wie Methanol, Äthanol, Propanol, Butanol, Methoxyäthanol, Äthoxyäthanol, Amide, wie Dimethylformamid, Dimethylacetamid, Tetramethylharnstoff, N-Methylpyrrolidon, Hexamethylphosphorsäuretriamid, Dimethylsulfoxid, Ketone, wie Aceton, Methyläthylketon, Cyclohexanon, Halogenkohlenwasserstoffe, wie Methylenchlorid, Chloroform, Dichloräthan, Äther, wie 1,2-Dimethoxyäthan, Tetrahydrofuran, Dioxan.

Die Umsetzungen nach Verfahren Aa) werden zweckmäßig in Gegenwart eines Kondensationsmittels durchgeführt. Als Kondensationsmittel kommen anorganische und organische Säurehalogenide in Frage, beispielsweise Thionylchlorid, Phosphortrichlorid, Phosphorpentachlorid, Phosphoroxychlorid, Chlorsulfonsäure, Phosgen, Oxalylchlorid, Chlorameisensäurealkylester, Benzoylchloride, Benzolsulfosäurechlorid oder 4-Toluolsulfosäurechlorid.

Werden bei der Umsetzung nach Verfahren Aa) Carbonsäurethioamide bzw. Thiolactame eingesetzt, so empfiehlt sich die Mitverwendung eines schwefelbindenden Mittels. Als schwefelbindende Mittel kommen beispielsweise Schwermetalloxide, wie Quecksilberoxid und Bleioxid in Frage.

Die Reaktionskomponenten nach Verfahren Aa) werden zweckmäßig in äquimolaren Mengen zur Umsetzung gebracht. Die drei letztgenannten Komponenten, insbesondere die Carbonsäureamide und Thioamide, Lactame und Thiolactame, können mit Vorteil auch im Überschuß angewendet werden.

Die Umsetzungen nach Verfahren A) d) zu Imidoäthern der Formel VII, und A) e) zu Trichloräthylidenverbindungen der Formel IX werden zweckmäßig in Gegenwart eines Kondensationsmittels durchgeführt. Als Kondensationsmittel kommen z. B. in Frage:
Essigsäureanhydrid, Polyphosphorsäure, Phosphorpentoxid, konz. Schwefelsäure. Im allgemeinen genügen katalytische Mengen der Kondensationsmittel.

Die Umsetzungen nach Verfahren A) f) und A) g) zu Amiden der Formel X können auch in Gegenwart von säurebindenden Mitteln durchgeführt werden. Als säurebindende Mittel kommen in Frage schwache Basen wie Triäthylamin oder Pyridin.

In den Verfahrensvarianten B) a) und B) b) empfiehlt sich bei Verwendung der freien Phenole der Formel XVI die Anwendung eines säurebindenden Mittels. Als säurebindende Mittel kommen Basen wie Triäthylamin oder Pyridin, sowie Alkali- und Erdalkalicarbonate und -bicarbonate, -hydroxide und -alkoxide, wie z. B. -methoxide, -äthoxide, -butoxide in Frage.

Die Reaktionstemperaturen liegen je nach Verfahrensvariante zwischen 0 und 200°C, vorzugsweise bei
Verfahren A) a) zwischen 0 und 50°C,
Verfahren A) b) zwischen 50 und 150°C,
Verfahren A) c) zwischen 100 und 150°C,
Verfahren A) d) zwischen 100 und 150°C,
(Vorprodukt der Formel VII bei 50 - 100°C)
Verfahren A) e) zwischen 100 und 150°C,
(Vorprodukt der Formel IX bei 50 - 100°C)
Verfahren A) f) zwischen 50 und 100°C,
A) g) zwischen 50 und 100°C (Vorprodukt der Formel X bei 50 - 100°C)
Verfahren B) a) zwischen 0 und 50C,
Verfahren B) b) zwischen 100 und 150°C.

Die Reaktionsbedingungen bei den einzelnen Stufen von Verfahren C) sind folgende:

| Verfahren C | | Reaktionsmdium | Temperaturen |
|---|---|---|---|
| Darstellung von | XXII | wie bei A) a) u. A) b) | 50 - 150°C |
| " " | XXIII | wie bei A) c) | 25 - 100°C |
| " " | XVIII | wie bei A) a) | 0 - 50°C |
| " " | I | wie bei A) c) | 50 - 150°C |

Die Reaktionszeiten betragen je nach Verfahrenvariante und je nach Temperaturbereich wenige Minuten bis einige Stunden.

Die Reaktionsprodukte nach Verfahren A) a) fallen in Form ihrer Salze an. Sie können als solche isoliert werden, oder gegebenenfalls durch Alkalischstellen der wäßrigen Lösungen in die freien Basen übergeführt werden.

Fallen die freien Basen in fester Form an, so ist erforderlichenfalls eine Reinigung durch Umkristallisation aus einem geeigneten Lösungsmittel oder Lösungsmittelgemisch möglich.

Zum Alkalischstellen benutzt man üblicherweise starke Basen, wie Ammoniak, Soda, Pottasche, Ätznatron, Ätzkali oder ihre wäßrigen Lösungen. Die freigesetzten Basen können wiederum mit physiologisch verträglichen Säuren in Salze überführt werden.

Die nach den beschriebenen Verfahrensvarianten erhaltenen Sulfide der Formel I (Q = -S-) können durch Oxydation in entsprechende Sulfoxide (Q = -SO-) bzw. Sulfone (Q = $-SO_2$) übergeführt werden. Die Oxydation wird durch Einwirkung von einem bzw. zwei Moläquivalent eines Oxydationsmittels durchgeführt. Als Oxydationsmittel kommen beispielsweise in Frage Wasserstoffperoxid oder Persäuren, wie z.B. Perameisensäure, Peressigsäure, Perbenzoesäure, 3-Chlorperbenzoesäure,

ferner Jodsäure, Perjodsäure, Chromsäure und ihre Salze bzw. Komplexe. Die Oxydationsreaktion wird im allgemeinen bei Temperaturen zwischen 0 und 100°C, bevorzugt zwischen 25 und 60°C durchgeführt.

Als physiologisch verträgliche Säuren kommen beispielsweise in Frage Halogenwasserstoffsäuren, insbesondere Salzsäure, ferner Schwefelsäure, Salpetersäure, Phosphorsäure, Essigsäure, Propionsäure, Milchsäure, Weinsäure, Zitronensäure, Oxalsäure, Bernsteinsäure, Maleinsäure,Fumarsäure, Sorbinsäure, Salicylsäure, Methyl-, Phenyl-, 4-Tolysulfonsäure oder 1,5-Naphthalindisulfonsäure.

Erforderlichenfalls kann eine Reinigung der Verfahrenserzeugnisse durch Umkristallisation aus einem geeigneten Lösungsmittel oder Lösungsmittelgemisch oder durch Säulenchromatografie über Kieselgel erfolgen.

Aus der allgemeinen Formel (I) ist ersichtlich, daß die erfindungsgemäßen Verbindungen mindestens zwei asymmetrische Kohlenstoffatome aufweisen, die sich in 2- und 4-Stellung des Dioxolanringes befinden. Diese Verbindungen können demnach in Form verschiedener Stereoisomeren vorliegen.

Die diasteromeren Racemate (cis- oder trans-Form) der Verbindungen der allgmeinen Formel I können in üblicher Weise, z. B. durch selektive, fraktionierte Kristallisation oder Säulenchromatographie getrennt werden.

Da die stereochemische Konfiguration bereits in den Zwischenprodukten der allgemeinen Formel (II) vorgegeben ist, kann die Trennung in die cis- und trans-Form bereits auf dieser Stufe oder bevorzugt noch früher, beispielsweise auf der Stufe der Zwischenprodukte der allgemeinen Formel XVII, XIX, XXI, XXII erfolgen.

Die cis- und trans-diasteromeren Racemate können ihrerseits in üblicher Weise in ihre optischen Antipoden cis(+), cis(-) bzw. trans(+) und trans(-) getrennt werden.

Die erfindungsgemäßen Verbindungen der Formel (I), insbesondere die als Racemate vorliegenden cis-Verbindungen, sind Chemotherpeutika und besitzen eine dem eingangs erwähnten Ketoconazol überlegene Wirkung und Verträglichkeit gegen Pilzinfektionen. Sie sind in vitro sehr gut wirksam gegen Hautpilze, wie z. B. Trichophyton mentagrophytes, Microsporum canis, Schimmelpilze, wie z. B. Aspergillus niger oder Hefen, wie z. B. Candida albicans, C. tropicalis. Die Verbindungen der Formel I wirken außerdem gegen Bakterien und Protozoen.

Auch in vivo, z. B. bei der experimentellen Nierencandidose der Maus, besitzen die Verbindungen sowohl nach oraler als auch nach parenteraler Anwendung einen sehr guten systemischen Effekt, z. B. gegen Candida albicans. Ebenso besteht ein sehr guter Effekt gegen verschiedene Erreger die Hautmykosen wie Trichophyton metagrophytes am Meerschweinchen. Die erfindungsgemäßen Verbindungen der Formel I wirken demnach sowohl nach oraler als auch nach parenteraler oder lokaler Applikation. Sie eignen sich dementsprechend zur Behandlung von Haut-Mykosen, System-Mykosen, insbesondere Candida-Mykosen.

Herstellungsbeispiele für Verbindungen der Formel I mit Q gleich Sauerstoff, $R^{1'}$ und $R^{2'}$ gleich Wasserstoff

Beispiel 1 (Verfahren A a)

Beispiel 1.1)

cis-2-(Imidazolyl-1-methyl)-2-(2,4-dichlorphenyl)-1,3-dioxolan-4-yl-[4-methoxy-(morpholinomethylen-anilin)]

Zu 29 g = 25,2 ml N-Formyl-morpholin (Mol.-Gew. 115) werden unter Rühren und Kühlung bei 25 - 28°C 1,85 g = 1,14 ml Phosphoroxichlorid (Mol.-Gew. 154) zugetropft und danach noch 2 Stunden bei Raumtemperatur gerührt. Dann werden unter Rühren und Kühlung bei 25 - 28°C 4,2 g cis-2-(Imidazolyl-1-methyl)-2-(2,4-dichlorphenyl)-1,3-dioxolan-4-yl-)4-methoxy-anilin) (Mol.-Gew. 420, Lit.-Fp. 160°C) portionsweise hinzugefügt. Die Reaktionslösung wird 2 Stunden bei Raumtemperatur gerührt, in 300 ml Äther eingerührt und der in Äther gelöste N-Formyl-morpholin-Überschuß (zur Wiedergewinnung) abdekandiert. Der halbfeste Rückstand wird in 150 ml Wasser gelöst, Aktivkohle zugesetzt, über eine Klärschicht abgesaugt, mit 150 ml Methylenchlorid versetzt und die wäßrige Lösung mit 2n-Ammoniak alkalisch gestellt. Das Endprodukt wird 2 x mit Methylenchlorid ausgeschüttelt, die vereinigten Methylenchloridphasen über Natriumsulfat getrocknet, eingedampft und der ölige Rückstand unter Zugabe von Petroläther/Isopropanol zur Kristallisation gebracht.

Man erhält so 4,5 g = 87 % der Theorie cis-2-(Imidazolyl-1-methyl)-2-(2,4-dichlorphenyl)-1,3-dioxolan-4-yl-/4-methoxy-(morpholinommethylen-anilin)/ in Form eines weißen, kristallinen Pulvers von Fp. 137°C.

Das als Ausgangsstoff verwendete cis-2-(Imidazolyl-1-methyl)-2-(2,4-dichlorphenyl)-1,3-dioxolan-4-yl-(4-methoxy-anilin) wird nach dem in DOS 2.804.096 beschriebenen Verfahren oder nach dem auf Seite 22 beschriebenen Verfahren dargestellt.

cis-2-(Imidazolyl-1-methyl)-2-(2,4-dichlorphenyl)-1,3-dioxolan-4-yl-(4-methoxy-anilin)

45,0 g (0,1 Mol) cis-2-(Imidazolyl-1-methyl)-2-(2,4-dichlorphenyl)-1,3-dioxolan-4-yl-(4-methoxy-nitrobenzol) (Fp. 136°C) werden in 500 ml Äthanol gelöst, 12,0 g (0,11 Mol) Calciumchlorid gelöst in 50 ml Wasser und 100 ml Äthanol zugegeben und unter gutem Rühren bei 25°C (leicht exotherme Reaktion!) 32,5 g (0,5 Mol) Zink-Staub portionsweise eingetragen. Ist alles eingetragen, wird die Reduktionsmischung 2 Stunden unter Rückfluß gerührt. Nach dem Abkühlen, wird vom unverbrauchten Zink-Staub abfiltriert und die Lösung am Rotationsverdampfer unter vermindertem Druck eingedampft. Der Rückstand wird aus Toluol umkristallisiert. Man erhält so 33,0 g = 79 % der Theorie cis-2-(Imidazolyl-1-methyl)-2-(2,4-dichlorphenyl)-1,3-dioxolan-4-yl-(4-methoxy-anilin) in Form von weißen Kristallen mit dem Fp. 160°C.
Die Substanz ist identisch mit der nach DOS 2.804.096 erhaltenen Verbindung und rein genug für weitere Umsetzungen.

Die als Ausgangsstoff verwendete Nitroverbindung wird wie folgt dargestellt:

cis-2-(Imidazolyl-1-methyl)-2-(2,4-dichlorphenyl)-1,3-dioxolan-4-yl-(4-methoxy-nitrobenzol)

46,3 g (0,1 Mol) cis-2-(Brommethyl)-2-(2,4-dichlorphenyl)-1,3-dioxolan-4-yl-(4-methoxy-nitrobenzol) (Fp. 192°C) werden zusammen mit 20,4 g (0,3 Mol) Imidazol in 500 ml Dimethylformamid 3 Tage unter Rückfluß erhitzt. Die Reaktionsmischung wird am Rotationsverdampfer und vermindertem Druck eingedampft, der Imidazol-Überschuß durch Anrühren mit Wasser extrahiert, der Rückstand in Methylenchlorid gelöst und unter Zusatz von Methanol (20 : 1) über eine Säule beladen mit Kieselgel chromatographiert.

Nach Eindampfen der chromatographierten Lösung und Umkristallisation des Rückstandes aus wenig Äthanol erhält man 28,0 g = 62 % der Theorie cis-2-(Imidazolyl-1-methyl)-2-(2,4-dichlorphenyl)-1,3-dioxolan-4-yl-(4-methoxy-nitrobenzol) in Form von weißen Kristallen mit dem Fp. 136°C.

Die Substanz ist identisch mit der durch Umsetzung von cis-2-(Imidazolyl-1-methyl)-2-(2,4-dichlorphenyl)-1,3-dioxolan-4-yl-methanol-mesylat (nach DOS 2.804.096) mit 4-Nitrophenol-Natrium-Salz gewonnenen Verbindung.

Das als Ausgangsstoff verwendete cis-2-Brommethyl-2-(2,4-dichlorphenyl)-1,3-dioxolan-4-yl-(4-methoxy-nitrobenzol) wird wie unter Verfahren C beschrieben dargestellt.

Das nach Beispiel 1.1 hergestellte cis-2-(Imidazolyl-1-methyl)-2-(2,4-dichlorphenyl)-1,3-dioxolan-4-yl-(4-methoxy-morpholinomethylen-anilin) wird auch wir folgt erhalten:

Verfahren A b)

Durch Umsetzung von cis-2-(Imidazolyl-1-methyl)-2-(2,4-dichlorphenyl)-1,3-dioxolan-4-yl-(4-methoxy-anilin) mit N-Formyl-morpholin-diäthylacetal in Pyridin bei 50°C.

Verfahren A c)

Diese Verfahrensvariante ist zur Darstellung von cis-2-(Azolyl-1-methyl)-2-(subst.-phenyl)-1,3-dioxolan-4-yl-(4-methoxy-(monoalkylamino-methylen-anilinen) geeignet, wie sie in den Beispiel 1.1, 1.2, 1.3, 1.4, 1.5, 1.17, 1.18 genannt werden.

Man erhält diese Verbindungen durch Umsetzung von cis-2-(Imidazolyl-1-methyl)-2-(2,4-dichlorphenyl)-1,3-dioxolan-4-yl-(4-methoxy-anilin) mit entsprechenden Imidoäthern (Formel V) in Eisessig oder Dimethylformamid bei 120°C.

Verfahren A d)

Durch Umsetzung von cis-2-(Imidazolyl-1-methyl)-2-(2,4-dichlorphenyl)-1,3-dioxolan-4-yl-(4-methoxy-anilin) mit Orthoameisensäuretrimethylester ohne Verdünnungsmittel in Gegenwart von etwas Essigsäureanhydrid bei 100°C und anschließender Umsetzung des so gewonnenen Imidoäthers mit Morpholin ohne Verdünnungsmittel bei 100°C.

Verfahren A e)

Durch Umsetzung von cis-2-(Imidazolyl-1-methyl)-2-(2,4-dichlorphenyl)-1,3-dioxolan-4-yl-(4-methoxy-anilin) mit wasserfreiem Trichloracetaldehyd (Chloral) in Polyphosphorsäure in Gegenwart von Phosphorpentoxid bei 70°C und anschließender Umsetzung der so erhaltenen Trichloräthyliden-Verbindung mit Morpholin ohne Verdünnungsmittel bei 100°C.

Die Verfahren A d) und A e) werden vorteilhaft bei der Darstelllung der Piperazino-Verbindungen, wie sie in den Beispielen 1.14, 1.15, 1.16 genannt werden, angewendet.

Verfahren A f)

Diese Verfahrensvariante ist zur Darstellung von cyclischen Amidinen geeignet, wie sie in den Beispielen 1.26 und 1.27 genannt werden.

Man erhält diese Verbindungen durch Umsetzung von Imidchloriden (Formel XI) mit Aminen (Formel VIII) in Äthylenglycoldimethyläther bei 85°C.

Die Imidchloride werden durch Umsetzung der entsprechenden Amide (Formel X) mit Phosphorpentachlorid in Toluol bei 80°C erhalten.

Verfahren A g)

Die Verfahrensvariante ist zur Darstellung von Amidinen geeignet wie sie in den Beispielen 1.1 - 1.25 und 1.28 und 1.29 genannt werden.

Man erhält diese Verbindungen durch Umsetzung von Imidchloriden der Formel XIII mit primären Aminen der Formel XIV in Äthylenglycoldimethyläther bei 85°C.

Die Imidchloride werden durch Umsetzung der entsprechenden Amide der Formel XII mit Phosphorpentachlorid in Toluol bei 80°C erhalten.

Verfahren B a)

Durch Umsetzung von 5,14 g (0,01 Mol) cis-2-(Imidazolyl-1-methyl)-2-(2,4-dichlorphenyl)-1,3-dioxolan-4-methanol-(4-nitrobenzolsulfonsäureester) (Formel XV) mit 2,06 g (0,01 Mol) 4-Morpholinomethylenimino-phenol (Formel XVI) in Gegenwart von 1,38 g (0,01 Mol) Kaliumcarbonat in Dimethylformamid bei 40°C erhält man die Verbindung nach Beispiel 1.1 mit indentischem Fp. 137°C. in 56 %iger Ausbeute.

Den als Ausgangsstoff verwendeten 4-Nitrobenzolsulfonsäureester erhält man durch Umsetzung von molaren Mengen cis-2-(Imidazolyl-1-methyl)-2-(2,4-dichlorphenyl)-1,3-dioxolan-4-methanol mit 4-Nitrobenzolsulfonsäurechlorid in Gegenwart von Triäthylamin.

4-Morpholinomethylenimino-phenol erhält man durch saure Spaltung des entsprechenden Phenol-O-benzyläthers. Dieser wird dargestellt durch Umsetzung von 4-Benzyloxy-anilin mit N-Formyl-morpholin in Gegenwart von Phosphoroxichlorid bei 40°C.

Verfahren B b)

Durch Umsetzung von 5,27 g (0,01 Mol) cis-2-Brommethyl-2-(2,4-dichlorphenyl)-1,3-dioxolan-4-methanol-(4-nitrobenzolsulfonsäureester) (Formel XVII) mit 2,06 g (0,01 Mol) 4-Morpholinomethylenimino-phenol (Formel XVI) in Gegenwart von 1,38 g (0,01 Mol) Kalciumcarbonat in Dimethylformamid bei 40°C erhält man cis-2-Brommethyl-2-(2,4-dichlorphenyl)-1,3-dioxolan-4-yl-/4-methoxy-(morpholinomethylen-anilin)7 in 78 %iger Ausbeute, welches mit überschüssigem Imidazol in Dimethylformamid bei 100°C (24 Stunden Rückfluß) zur Verbindung nach Beispiel 1.1 mit indentischem Fp. 137°C mit 45 %iger Ausbeute führt.

Den als Ausgangsstoff verwendeten 4-Nitrobenzolsulfonsäureester erhält man durch Umsetzung von molaren Mengen cis-2-Brommethyl-2-(2,4-dichlorphenyl)-1,3-dioxolan-4-methanol mit 4-Nitrobenzolsulfonsäurechlorid in Gegenwart von Pyridin.

Verfahren C)

Beispiel 1.2

cis-2-(Imidazolyl-1-methyl)-2-(2,4-dichlorphenyl)-1,3-dioxolan-4-yl-/4-methoxy-(morpholinomethylen-anilin)7

5,3 g (0,01 Mol cis-2-Brommethyl-2-(2,4-dichlorphenyl)-1,3-dioxolan-4-yl-/4-methoxy-(morpholinomethylen-anilin)7 werden zusammen mit 2,0 g (0,03 Mol) Imidazol in 50 ml Dimethylformamid 3 Tage unter Rückfluß erhitzt und wie unter Verfahren A) a) beschrieben aufgearbeitet. Die so erhaltene Titelverbindung ist identisch (Fp. 137°C) mit der nach Verfahren A) a) erhaltenen.

Das als Ausgangsstoff verwendete cis-2-Brommethyl-2-(2,4-dichlorphenyl)-1,3-dioxolan-4-yl-/4-methoxy-(morpholinomethylen-anilin)7 wird dargestellt durch Umsetzung von 4,3 g

(0,01 Mol) cis-2-Brommethyl-2-(2,4-dichlorphenyl)-1,3-dioxolan- 4-yl-(4-methoxy-anilin) und dem Vilsmeier-Komplex aus 29,0 g N-Formyl-morpholin (Mol.-Gew. 115) und 1,85 g Phosphoroxichlorid (Mol.-Gew. 154), 2 Stunden 25°C wie unter Verfahren A) a) beschrieben.

Das hierbei als Ausgangsstoff verwendete cis-2-Brommethyl-2-(2,4-dichlorphenyl)-1,3-dioxolan-4-yl-(4-methoxy-anilin) wird dargestellt durch Reduktion von 4,63 g (0,01 Mol) cis-2-Brommethyl-2-(2,4-dichlorphenyl)-1,3-dioxolan-4-yl-(4-methoxy-nitrobenzol) mittels 3,25 g (0,05 Mol) Zink-Staub und 1,2 g (0,011 Mol) Calciumchlorid in siedendem Äthanol.

Das als Ausgangsstoff verwendete cis-2-(Brommethyl-2-(2,4-dichlorphenyl)-1,3-dioxolan-4-yl-(4-methoxy-nitrobenzol) wird wie folgt dargestellt:

26,8 (0,1 Mol) ω-Brom-2,4-dichloracetophenon, 21,3 (0,1 Mol) α-Glycerin-4-nitrophenyläther und 1 g 4-Toluolsulfonsäure (als Kondensationskatalysator) werden in 50 ml n-Butanol und 150 ml Toluol gelöst und 12 Stunden unter Rückfluß am Wasserabscheider erhitzt. Sodann wird die Reaktionsmischung am Rotationsverdampfer im Vakuum eingedampft, der Rückstand in Methylenchlorid aufgenommen, zweimal mit 2n-Sodalösung ausgeschüttelt, die Methylenchloridphase abgetrennt, über Natriumsulfat getrocknet, eingedampft und der Rückstand aus Toluol umkristallisiert.

Man erhält so 24 g = 52 % der Theorie cis-2-Brommethyl-2-(2,4-dichlorphenyl)-1,3-dioxolan-4-yl-(4-methoxy-nitrobenzol) als weißes Pulver mit dem Fp. 192°C.

Der als Ausgangsstoff verwendete α-Glycerin-4-nitrophenyläther wird durch Umsetzung von molaren Mengen 3-Chlor-1,2-dihydroxypropan und 4-Nitrophenol-Natriumsalz in abs. Äthanol 24 Stunden Rückfluß in fast quantitativer Ausbeute hergestellt. Die Verbindung ist ein gelbes Öl.

Nach den in Beispiel 1.1 beschriebenen Verfahren werden erhalten:

1.3) Cis-2-(Imidazolyl-1-methyl)-2-(2,4-dichlorphenyl)-1,3-dioxolan-4-yl-[4-methoxy-(aminomethylen-anilin)] aus Cis-2-(Imidazolyl-1-methyl)-2-(2,4-dichlorphenyl)-1,3-dioxolan-4-yl-(4-methoxy-anilin) = DMOA und Formamid

1.4) Cis-2-(Imidazolyl-1-methyl)-2-(2,4-dichlorphenyl)-1,3-dioxolan-4-yl-[4-methoxy-(n-methylaminomethylen-anilin)] aus DMOA und N-Methyl-formamid

1.5) Cis-2-(Imidazolyl-1-methyl)-2-(2,4-dichlorphenyl)-1,3-dioxolan-4-yl-[4-methoxy-(N-äthylaminomethylen-ainilin)] aus DMOA und N-Äthyl-formamid

1.6) Cis-2-(Imidazolyl-1-methyl)-2-(2,4-dichlorphenyl)-1,3-dioxolan-4-yl-[4-methoxy-(N-n-butylaminomethylen-anilin)] aus DMOA und N-n-Butyl-formamid

1.7) Cis-2-(Imidazolyl-1-methyl)-2-(2,4-dichlorphenyl)-1,3-dioxolan-4-yl-[4-methoxy-(N-dimethylaminomethylen-anilin)] Fp. 135°C aus DMOA und N-Dimethyl-formamid

1.8) Cis-2-(Imidazolyl-1-methyl)-2-(2,4-dichlorphenyl)-1,3-dioxolan-4-yl-[4-methoxy-(N-diäthylaminomethylen-anilin)] aus DMOA und N-Diäthyl-formamid

1.9) Cis-2-(Imidazolyl-1-methyl)-2-(2,4-dichlorphenyl)-1,3-dioxolan-4-yl-[4-methoxy-(N-di-n-propylaminomethylen-anilin)] aus DMOA und N-Di-n-propyl-formamid

1.10) Cis-2-(Imidazolyl-1-methyl)-2-(2,4-dichlorphenyl)-1,3-dioxolan-4-yl-[4-methoxy-(N-di-n-butylaminomethylen-anilin)] aus DMOA und N-Di-n-butyl-formamid

0094052

1.11) Cis-2-(Imidazolyl-1-methyl)-2-(2,4-dichlorphenyl)-1,3-dioxolan-4-yl-/4-methoxy-(pyrrolidinomethylen-anilin)7 aus DMOA und N-Formyl-pyrrolidin

1.12) Cis-2-(Imidazolyl-1-methyl)-2-(2,4-dichlorphenyl)-1,3-dioxolan-4-yl-/4-methoxy-(piperidinomethylen-anilin)7 aus DMOA und N-Formyl-piperidin

1.13) Cis-2-(Imidazolyl-1-methyl)-2-(2,4-dichlorphenyl)-1,3-dioxolan-4-yl-/4-methoxy-(2,6-dimethylmorpholinomethylen-anilin aus DMOA uns N-Formyl-2,6-dimethylmorpholin

1.14) Cis-2-(Imidazolyl-1-methyl)-2-(2,4-dichlorphenyl)-1,3-dioxolan-4-yl-/4-methoxy-(thiomorpholinomethylen-anilin)7 Fp. 130°C aus DMOA und N-Formyl-thiomorpholin

1.15) Cis-2-(Imidazolyl-1-methyl)-2-(2,4-dichlorphenyl)-1,3-dioxolan-4-yl-/4-methoxy-(N-Methylpiperazinomethylen-anilin)7 aus DMOA und N-Formyl-N'-methyl-piperazin

1.16) Cis-2-(Imidazolyl-1-methyl)-2-(2,4-dichlorphenyl)-1,3-dioxolan-4-yl-/4-methoxy-(N-Benzylpliperazinomethylen-anilin)7 aus DMOA und N-Formyl-N'-benzyl-piperazin

1.17) Cis-2-(Imidazolyl-1-methyl)-2-(2,4-dichlorphenyl)-1,3-dioxolan-4-yl-/4-methoxy-(N-Phenylpiperazinomethylen-anilin)7 aus DMOA und N-Formyl-N'-phenyl-piperazin

1.18) Cis-2-(Imidazolyl-1-methyl)-2-(2,4-dichlorphenyl)-1,3-dioxolan-4-yl-/4-methoxy-(amino-1-äthylen-anilin)7 aus DMOA und Acetamid

1.19) Cis-2-(Imidazolyl-1-methyl)-2-(2,4-dichlorphenyl)-1,3-dioxolan-4-yl-/4-methoxy-(N-methylamino-1-äthylen-anilin)7 aus DMOA und N-Methyl-acetamid

1.20) Cis-2-(Imidazolyl-1-methyl)-2-(2,4-dichlorphenyl)-1,3-dioxolan-4-yl-/4-methoxy-(N-dimethylamino-1-äthylen-anilin)/ Fp. 120°C aus DMOA und N-Dimethyl-acetamid

1.21) Cis-2-(Imidazolyl-1-methyl)-2-(2,4-dichlorphenyl)-1,3-dioxolan-4-yl-/4-methoxy-(N-diäthylamino-1-äthylen-anilin)/ aus DMOA und N-Diäthyl-acetamid

1.22) Cis-2-(Imidazolyl-1-methyl)-2-(2,4-dichlorphenyl)-1,3-dioxolan-4-yl-/4-methoxy-(pyrrolidino-1-äthylen-anilin)/ aus DMOA und N-Acetyl-pyrrolidin

1.23) Cis-2-(Imidazolyl-1-methyl)-2-(2,4-dichlorphenyl)-1,3-dioxolan-4-yl-/4-methoxy-(piperidiono-1-äthylen-anilin)/ aus DMOA und N-Acetyl-piperidin

1.24) Cis-2-(Imidazolyl-1-methyl)-2-(2,4-dichlorphenyl)-1,3-dioxolan-4-yl-/4-methoxy-(morpholino-1-äthylen-anilin)/ aus DMOA und N-Acetyl-morpholin

1.25) Cis-2-(Imidazolyl-1-methyl)-2-(2,4-dichlorphenyl)-1,3-dioxolan-4-yl-/4-methoxy-(N-methylpiperazino-1-äthylen-anilin)/ aus DMOA und N-Acetyl-N'-methyl-piperazin

1.26) Cis-2-(Imidazolyl-1-methyl)-2-(2,4-dichlorphenyl)-1,3-dioxolan-4-yl-/4-methoxy-(N-diemthylamino-1-propylen-anilin)/ aus DMOA und N-Dimethyl-propionamid

1.27) Cis-2-(Imidazolyl-1-methyl)-2-(2,4-dichlorphenyl)-1,3-dioxolan-4-yl-/4-methoxy-(N-methyllpyrrolidon-2-imino-benzol)/ aus DMOA und N-Methyl-pyrrolidon-2

1.28) Cis-2-(Imidazolyl-1-methyl)-2-(2,4-dichlorphenyl)-1,3-dioxolan-4-yl-/4-methoxy-(N-methylpiperidon-2-imino-benzol)/ aus DMOA und N-Methyl-piperidon-2

1.29) Cis-2-(Imidazolyl-1-methyl)-2-(4-chlorphenyl)-1,3-dioxolan-4-yl-/4-methoxy-(morpholinomethylen-anilin)7 Fp. 160°C aus cis-2-(Imidazolyl-1-methyl)-2-(4-chlorphenyl)-1,3-dioxolan-4-yl-(4-methoxy-anilin) und N-Formyl-morpholin

1.30) Cis-2-(1, 2, 4-Triazolyl-1-methyl)-2-(2,4-dichlorphenyl)-1,3-dioxolan-4-yl-/4-methoxy-(morpholinomethylen-anilin)7 Fp. 150°C aus cis-2-(1,2,4-Triazolyl-1-methyl-2-(2,4-dichlorphenyl)-1,3-dioxolan-4-yl-(4-methoxy-anilin) und N-Formyl-morpholin

Wenn kein Schmelzpunkt angegeben ist, erfolgte die Identifizierung durch Massenspektrum und Kernresonanzsprektrum (NMR).

Herstellungsbeispiele für Verbindungen der Formel I mit Q= Sauerstoff und $R^{1'}/R^{2'}$ ungleich Wasserstoff

Beispiel 2 (Verfahren A a)

Beispiel 2.1)

cis-2-(Imidazolyl-1-methyl)-2-(2,4-dichlorphenyl)-1,3-dioxolan-4-yl-[2-methyl- 4-methoxy-(morpholinomethylen-anilin)]

Zu 29 g = 25,2 ml N-Formyl-morpholin (Mol.-Gew. 115) werden unter Rühren und Kühlung bei 25 - 28°C 1,85 g = 1,14 ml Phosphoroxichlorid (Mol.-Gew. 154) zugetropft und danach noch 2 Stunden bei Raumtemperatur gerührt. Dann werden unter Rühren und Kühlung bei 25 - 28°C 4,4 g cis-2-(Imidazolyl-1-methyl)-2-(2,4-dichlorphenyl)-1,3-dioxolan-4-yl-(2-methyl-4-methoxy-anilin) (Mol.-Gew. 434, zähes Öl), portionsweise eingetragen. Die Reaktionslösung wird 2 Stunden bei Raumtemperatur gerührt, in 300 ml Äther eingerührt und der in Äther gelöste N-Formyl-morpholin-Überschuß (zur Wiedergewinnung) abdekandiert. Der halbfeste Rückstand wird in 150 ml Wasser gelöst, Aktivkohle zugesetzt, über eine Klärschicht abgesaugt, mit 150 ml Methylenchlorid versetzt und die wäßrige Lösung mit 2n-Ammoniak alkalisch gestellt. Das Endprodukt wird 2 x mit Methylenchlorid ausgeschüttelt, die vereinigten Methylenchloridphasen über Natriumsulfat getrocknet, eingedampft und der ölige Rückstand unter Zugabe von Petroläther/Isopropanol zur Kristallisation gebracht.

Man erhält so 4 g = 75 % der Theorie cis-2-(Imidazolyl-1-methyl)-2-(2,4-dichlorphenyl)-1,3-dioxolan-4-yl-[2-methyl-4-methoxy-(morpholinommethylen-anilin)] in Form eines weißen, kristalinen Pulvers von Fp. 108°C.

Das als Ausgangsstoff verwendete cis-2-(Imidazolyl-1-methyl)-2-(2,4-dichlorphenyl)-1,3-dioxolan-4-yl-(2-methyl-4-methoxy-anilin) wird nach dem in DOS 2.804.096 beschriebenen Verfahren oder nach dem auf Seite 33 beschriebenen Verfahren dargestellt.

cis-2-(Imidazolyl-1-methyl)-2-(2,4-dichlorphenyl)-1,3-dioxolan-4-yl-(2-methyl-4-methoxy-anilin)

46,4 g (0,1 Mol) cis-2-(Imidazolyl-1-methyl)-2-(2,4-dichlorphenyl)-1,3-dioxolan-4-yl-(2-methyl-4-methoxy-nitrobenzol) (Fp. 108°C) werden in 500 ml Äthanol gelöst, 12,0 g (0,11 Mol) Calciumchlorid gelöst in 50 ml Wasser und 100 ml Äthanol zugegeben und unter gutem Rühren bei 25°C (leicht exotherme Reaktion!) 32,5 g (0,5 Mol) Zink-Staub portionsweise eingetragen. Ist alles eingetragen, wird die Reduktionsmischung 2 Stunden unter Rückfluß gerührt. Nach dem Abkühlen, wird vom unverbrauchten Zink-Staub abfiltriert und die Lösung am Rotationsverdampfer unter vermindertem Druck eingedampft. Der Rückstand wird aus Toluol umkristallisiert. Man erhält so 30 g = 69 % der Theorie cis-2-(Imidazolyl-1-methyl)-2-(2,4-dichlorphenyl)-1,3-dioxolan-4-yl-(2-methyl-4-methoxy-anilin) in Form eines zähen Öles.

Die als Ausgangsstoff verwendete Nitroverbindung wird wie folgt dargestellt:

cis-2-(Imidazolyl-1-methyl)-2-(2,4-dichlorphenyl)-1,3-dioxolan-4-yl-(2-methyl-4-methoxy-nitrobenzol)

47,7 g (0,1 Mol) cis-2-(Brommethyl)-2-(2,4-dichlorphenyl)-1,3-dioxolan-4-yl-(2-methyl-4-methoxy-nitrobenzol) (Fp. 162°C) werden zusammen mit 20,4 g (0,3 Mol) Imidazol in 500 ml Dimethylformamid 3 Tage unter Rückfluß erhitzt. Die Reaktionsmischung wird am Rotationsverdampfer und vermindertem Druck eingedampft, der Imidazol-Überschuß durch Anrühren mit Wasser extrahiert, der Rückstand in Methylenchlorid gelöst und unter Zusatz von Methanol (20 : 1) über eine Säule beladen mit Kieselgel chromatographiert.

Verfahren A d)

Durch Umsetzung von cis-2-(Imidazolyl-1-methyl)-2-(2,4-dichlorphenyl)-1,3-dioxolan-4-yl-(2-methyl-4-methoxy-anilin) mit Orthoameisensäuretrimethylester ohne Verdünnungsmittel in Gegenwart von etwas Essigsäureanhydrid bei 100°C und anschließender Umsetzung des so gewonnenen Imidoäthers mit Morpholin ohne Verdünnungsmittel bei 100°C.

Verfahren A e)

Durch Umsetzung von cis-2-(Imidazolyl-1-methyl)-2-(2,4-dichlorphenyl)-1,3-dioxolan-4-yl-(2-methyl-4-methoxy-anilin) mit wasserfreiem Trichloracetaldehyd (Chloral) in Polyphosphorsäure in Gegenwart von Phosphorpentoxid bei 70°C und anschließender Umsetzung der so erhaltenen Trichloräthyliden-Verbindung mit Morpholin ohne Verdünnungsmittel bei 100°C.

Die Verfahren A d) und A e) werden vorteilhaft bei der Darstelllung der Piperazino-Verbindungen, wie sie im Beispiel 2.7 genannt wird, angewendet.

Verfahren A f)

Diese Verfahrensvariante ist zur Darstellung von cyclischen Amidinen geeignet, wie sie im Beispiel 2.9 genannt wird.

Man erhält diese Verbindungen durch Umsetzung von Imidchloriden (Formel XI) mit Aminen (Formel VIII) in Äthylenglycoldimethyläther bei 85°C.

Die Imidchloride werden durch Umsetzung der entsprechenden Amide (Formel X) mit Phosphorpentachlorid in Toluol bei 80°C erhalten.

Nach Eindampfen der chromatographieten Lösung und Umkristallisation des Rückstandes aus wenig Äthanol erhält man 27 g = 58 % der Theorie cis-2-(Imidazolyl-1-methyl)-2-(2,4-dichlorphenyl)-1,3-dioxolan-4-yl-(2-methyl-4-methoxy-nitrobenzol) in Form von weißen Kristallen mit dem Fp. 108°C.

Die Substanz ist identisch mit der durch Umsetzung von cis-2-(Imidazolyl-1-methyl)-2-(2,4-dichlorphenyl)-1,3-dioxolan-4-yl-methanol-mesylat (nach DOS 2.804.096) mit 4-Nitro-3-methylphenol-Natrium-Salz gewonnenen Verbindung.

Das als Ausgangsstoff verwendete cis-2-Brommethyl-2-(2,4-dichlorphenyl)-1,3-dioxolan-4-yl-(2-methyl-4-methoxy-nitrobenzol) wird wie unter Verfahren C beschrieben dargestellt.

Das nach Beispiel 2.1 hergestellte cis-2-(Imidazolyl-1-methyl)-2-(2,4-dichlorphenyl)-1,3-dioxolan-4-yl-(2-methyl-4-methoxy-morpholinomethylen-anilin) wird auch wir folgt erhalten:

<u>Verfahren A b)</u>

Durch Umsetzung von cis-2-(Imidazolyl-1-methyl)-2-(2,4-dichlorphenyl)-1,3-dioxolan-4-yl-(2-methyl-4-methoxy-anilin) mit N-Formyl-morpholin-diäthylacetal in Pyridin bei 50°C.

<u>Verfahren A c)</u>

Diese Verfahrensvariante ist zur Darstellung von cis-2-(Azolyl-1-methyl)-2-(subst.-phenyl)-1,3-dioxolan-4-yl-(2-methyl-4-methoxy-(monoalkylamino-methylen-anilinen) geeignet.

Man erhält diese Verbindungen durch Umsetzung von cis-2-(Imidazolyl-1-methyl)-2-(2,4-dichlorphenyl)-1,3-dioxolan-4-yl-(2-methyl-4-methoxy-anilin) mit entsprechenden Imidoäthern (Formel V) in Eisessig oder Dimethylformamid bei 120°C.

Verfahren A g)

Die Verfahrensvariante ist zur Darstellung von Amidinen geeignet, wie sie in den Beispielen 2.1 - 2.8 und 2.10 - 2.16 genannt werden.

Man erhält diese Verbindungen durch Umsetzung von Imidchloriden der Formel XIII mit primären Aminen der Formel XIV in Äthylenglycoldimethyläther bei 85°C.

Die Imidchloride werden durch Umsetzung der entsprechenden Amide der Formel XII mit Phosphorpentachlorid in Toluol bei 80°C erhalten.

Verfahren B a)

Durch Umsetzung von 5,14 g (0,01 Mol) cis-2-(Imidazolyl-1-methyl)-2-(2,4-dichlorphenyl)-1,3-dioxolan-4-methanol-(4-nitrobenzolsulfonsäureester) (Formel XV) mit 2,2 g (0,01 Mol) 3-Methyl-4-morpholinomethylenimino-phenol (Formel XVI) in Gegenwart von 1,38 g (0,01 Mol) Kaliumcarbonat in Dimethylformamid bei 40°C erhält man die Verbindung nach Beispiel 2.1 mit indentischem Fp. 107°C. in 50 %iger Ausbeute.

Den als Ausgangsstoff verwendeten 4-Nitrobenzolsulfonsäureester erhält man durch Umsetzung von molaren Mengen cis-2-(Imidazolyl-1-methyl)-2-(2,4-dichlorphenyl)-1,3-dioxolan-4-methanol mit 4-Nitrobenzolsulfonsäurechlorid in Gegenwart von Triäthylamin.

3-Methyl-4-morpholinomethylenimino-phenol erhält man durch saure Spaltung des entsprechenden Phenol-O-benzyläthers. Dieser wird dargestellt durch Umsetzung von 4-Benzyloxy-2-methyl-anilin mit N-Formyl-morpholin in Gegenwart von Phosphoroxichlorid bei 40°C.

0094052

Verfahren B b)

Durch Umsetzung von 5,27 g (0,01 Mol) cis-2-Brommethyl-2-(2,4-dichlorphenyl)-1,3-dioxolan-4-methanol-(4-nitrobenzolsulfonsäureester) (Formel XVII) mit 2,2 g (0,01 Mol) 3-Methyl-4-morpholinomethylenimino-phenol (Formel XVI) in Gegenwart von 1,38 g (0,01 Mol) Kalciumcarbonat in Dimethylformamid bei 40°C erhält man cis-2-Brommethyl-2-(2,4-dichlorphenyl)-1,3-dioxolan-4-yl-/2-methyl-4-methoxy-(morpholinomethylen-anilin)/ in 78 %iger Ausbeute, welches mit überschüssigem Imidazol in Dimethylformamid bei 100°C (24 Stunden Rückfluß) zur Verbindung nach Beispiel 1.1 mit indentischem Fp. 107°C mit 50 %iger Ausbeute führt.

Den als Ausgangsstoff verwendeten 4-Nitrobenzolsulfonsäureester erhält man durch Umsetzung von molaren Mengen cis-2-Brommethyl-2-(2,4-dichlorphenyl)-1,3-dioxolan-4-methanol mit 4-Nitrobenzolsulfonsäurechlorid in Gegenwart von Pyridin.

Verfahren C)

Beispiel 2.2

cis-2-(Imidazolyl-1-methyl)-2-(2,4-dichlorphenyl)-1,3-dioxolan-4-yl-/2-methyl-4-methoxy-(morpholinomethylen-anilin)/

5,4 g (0,01 Mol cis-2-Brommethyl-2-(2,4-dichlorphenyl)-1,3-dioxolan-4-yl-/2-methyl-4-methoxy-(morpholinomethylen-anilin)/ werden zusammen mit 2,0 g (0,03 Mol) Imidazol in 50 ml Dimethylformamid 3 Tage unter Rückfluß erhitzt und wie unter Verfahren A) a) beschrieben aufgearbeitet. Die so erhaltene Titelverbindung ist identisch (Fp. 107°C) mit der nach Verfahren A) a) erhaltenen.

Das als Ausgangsstoff verwendete cis-2-Brommethyl-2-(2,4-dichlorphenyl)-1,3-dioxolan-4-yl-/2-methyl-4-methoxy-(morpholinomethylen-anilin)/ wird dargestellt durch Umsetzung von

4,4 g (0,01 Mol) cis-2-Brommethyl-2-(2,4-dichlorphenyl)-1,3-dioxolan- 4-yl-(2-methyl-4-methoxy-anilin) und dem Vilsmeier-Komplex aus 29,0 g N-Formyl-morpholin (Mol.-Gew. 115) und 1,85 g Phosphoroxichlorid (Mol.-Gew. 154), 2 Stunden 25°C wie unter Verfahren A) a) beschrieben.

Das hierbei als Ausgangsstoff verwendete cis-2-Brommethyl-2-(2,4-dichlorphenyl)-1,3-dioxolan-4-yl-(2-methyl-4-methoxy-anilin) wird dargestellt durch Reduktion von 4,77 g (0,01 Mol) cis-2-Brommethyl-2-(2,4-dichlorphenyl)-1,3-dioxolan-4-yl-(2-methyl-4-methoxy-nitrobenzol) mittels 3,25 g (0,05 Mol) Zink-Staub und 1,2 g (0,011 Mol) Calciumchlorid in siedendem Äthanol.

Das als Ausgangsstoff verwendete cis-2-(Brommethyl-2-(2,4-dichlorphenyl)-1,3-dioxolan-4-yl-(2-methyl-4-methoxy-nitrobenzol) wird wie folgt dargestellt:

26,8 (0,1 Mol) α-Brom-2,4-dichloracetophenon, 21,3 (0,1 Mol) α-Glycerin-4-nitro-3-methylphenyläther und 1 g 4-Toluolsulfonsäure (als Kondensationskatalysator) werden in 50 ml n-Butanol und 150 ml Toluol gelöst und 12 Stunden unter Rückfluß am Wasserabscheider erhitzt. Sodann wird die Reaktionsmischung am Rotationsverdampfer im Vakuum eingedampft, der Rückstand in Methylenchlorid aufgenommen, zweimal mit 2n-Sodalösung ausgeschüttelt, die Methylenchloridphase abgetrennt, über Natriumsulfat getrocknet, eingedampft und der Rückstand aus Toluol umkristallisiert.

Man erhält so 25 g = 50 % der Theorie cis-2-Brommethyl-2-(2,4-dichlorphenyl)-1,3-dioxolan-4-yl-(2-methyl-4-methoxy-nitrobenzol) als weißes Pulver mit dem Fp. 162°C.

Der als Ausgangsstoff verwendete α-Glycerin-4-nitro-3-methylphenyläther wird durch Umsetzung von molaren Mengen 3-Chlor-1,2-dihydroxypropan und 4-Nitro-3-methylphenol-Natriumsalz in abs. Äthanol 24 Stunden Rückfluß in fast quantitativer Ausbeute hergestellt. Die Verbindung ist ein gelbes Öl.

Nach den in Beispiel 2.2 beschriebenen Verfahren werden erhalten:

2.3) Cis-2-(Imidazolyl-1-methyl)-2-(2,4-dichlorphenyl)-1,3-dioxolan-4-yl-/2-methyl-4-methoxy-(N-dimethylamino-methylenanilin)7 Fp. 105°C) aus Cis-2-(Imidazolyl-1-methyl)-2-(2,4-dichlorphenyl)-1,3-dioxolan-4-yl-(2-methyl-4-methoxy-anilin) = M und N-Dimethylformamid.

2.4) Cis-2-(Imidazolyl-1-methyl)-2-(2,4-dichlorphenyl)-1,3-dioxolan-4-yl-/2-methyl-4-methoxy-(N-diäthylamino-methylenanilin)7 Öl, (Fp. 100°C) aus M und N-Diäthyl-formamid.

2.5) Cis-2-(Imidazolyl-1-methyl)-2-(2,4-dichlorphenyl)-1,3-dioxolan-4-yl-/2-methyl-4-methoxy-(pyrrolidinomethylen-anilin)7Öl, (Fp. 100°C) aus M und N-Formyl-pyrrolidin.

2.6) Cis-2-(Imidazolyl-1-methyl)-2-(2,4-dichlorphenyl)-1,3-dioxolan-4-yl-/2-methyl-4-methoxy-(piperidinomethylen-anilin)7 Öl, (Fp. 100°C) aus M und N-Formyl-piperidin.

2.7) Cis-2-(Imidazolyl-1-methyl)-2-(2,4-dichlorphenyl)-1,3-dioxolan-4-yl-/2-methyl-4-methoxy-(N-methylpiperazino methylen-anilin)7 aus M und N-Formyl-N'-methyl-piperazin.

2.8) Cis-2-(Imidazolyl-1-methyl)-2-(2,4-dichlorphenyl)-1,3-dioxolan-4-yl-/2-methyl-4-methoxy-(N-dimethylamino-1-äthylen-anilin)7 Öl, (Fp. 100°C) aus M und N-Dimethyl-acetamid.

2.9) Cis-2-(Imidazolyl-1-methyl)-2-(2,4-dichlorphenyl)-1,3-dioxolan-4-yl-/2-methyl-4-methoxy-(N-methylpyrrolidon-2-imino-benzol)7Öl, (Fp. 100°C) aus M und N-Methyl-pyrrolidon-2.

2.10 ) Cis-2-(Imidazolyl-1-methyl)-2-(2,4-dichlorphenyl)-1,3-dioxolan-4-yl-/3-methyl-4-methoxy-(N-dimethylamino-methylenanilin)7 Fp. 103°C
aus Cis-2-(Imidazolyl-1-methyl)-2-(2,4-dichlorphenyl)-1,3-dioxolan-4-yl-(3-methyl-4-methoxy-anilin) = U und N-Dimethylformamid.

2.11 ) Cis-2-(Imidazolyl-1-methyl)-2-(2,4-dichlorphenyl)-1,3-dioxolan-4-yl-/3-methyl-4-methoxy-(morpholinoemethylen-anilin)7 Fp. 108°C aus U und N-Formyl-morpholin.

2.12 ) Cis-2-(Imidazolyl-1-methyl)-2-(2,4-dichlorphenyl)-1,3-dioxolan-4-yl-/2-chlor-4-methoxy-(N-dimethylamino-methylenanilin)7 Fp. 121°C
aus Cis-2-(Imidazolyl-1-methyl)-2-(2,4-dichlorphenyl)-1,3-dioxolan-4-yl-(2-chlor-4-methoxy-anilin) = W und N-Dimethylformamid.

2.13 ) Cis-2-(Imidazolyl-1-methyl)-2-(2,4-dichlorphenyl)-1,3-dioxolan-4-yl-/2-chlor-4-methoxy-(morpholinomethylen-anilin)7 Fp. 123°C aus W und N-Formyl-morpholin.

2.14 ) Cis-2-(Imidazolyl-1-methyl)-2-(2,4-dichlorphenyl)-1,3-dioxolan-4-yl-/2,4-dimethoxy-(morpholinomethylen-anilin)7 Fp. 113°C
aus Cis-2-(Imidazolyl-1-methyl)-2-(2,4-dichlorphenyl)-1,3-dioxolan-4-yl-(2,4-dimethoxy-anilin) und N-Formyl-morpholin.

2.15 ) Cis-2-(Imidazolyl-1-methyl)-2-(2,4-dichlorphenyl)-1,3-dioxolan-4-yl-/2,6-dimethyl-4-methoxy-(morpholino-methylenanilin)7
aus Cis-2-(Imidazolyl-1-methyl)-2-(2,4-dichlorphenyl)-1,3-dioxolan-4-yl-(2,6-dimethyl-4-methoxy-anilin) und N-Formyl-morpholin.

0094052

2.16) Cis-2-(Imidazolyl-1-methyl)-2-(2,4-dichlorphenyl)-1,3-dioxolan-4-yl-/2,5-dimethyl-4-methoxy-(morpholinomethylenanilin)/
aus Cis-2-(Imidazolyl-1-methyl)-2-(2,4-dichlorphenyl)-1,3-dioxolan-4-yl-(2,5-dimethyl-4-methoxy-anilin) und N-Formylmorpholin.

Wenn kein Schmelzpunkt angegeben ist, erfolgte die Identifizierung durch Massenspektrum und Kernresonanzspektrum (NMR).

Herstellungsbeispiele für Verbindungen der Formel I mit Q ungleich Sauerstoff

Beispiel 3 (Verfahren A a)

Beispiel 3.1)

cis-2-(Imidazolyl-1-methyl)-2-(2,4-dichlorphenyl)-1,3-dioxolan-4-yl-[4-methylthio-(morpholinomethylen-anilin)]

Zu 29 g = 25,2 ml N-Formyl-morpholin (Mol.-Gew. 115) werden unter Rühren und Kühlung bei 25 - 28°C 1,85 g = 1,14 ml Phosphoroxichlorid (Mol.-Gew. 154) zugetropft und danach noch 2 Stunden bei Raumtemperatur gerührt. Dann werden unter Rühren und Kühlung bei 25 - 28°C 4,4 g cis-2-(Imidazolyl-1-methyl)-2-(2,4-dichlorphenyl)-1,3-dioxolan-4-yl-(4-methylthio-anilin) (Mol.-Gew. 436, zähes Öl), portionsweise hinzugefügt. Die Reaktionslösung wird 2 Stunden bei Raumtemperatur gerührt, in 300 ml Äther eingerührt und der in Äther gelöste N-Formyl-morpholin-Überschuß (zur Wiedergewinnung) abdekandiert. Der halbfeste Rückstand wird in 150 ml Wasser gelöst, Aktivkohle zugesetzt, über eine Klärschicht abgesaugt, mit 150 ml Methylenchlorid versetzt und die wäßrige Lösung mit 2n-Ammoniak alkalisch gestellt. Das Endprodukt wird 2 x mit Methylenchlorid ausgeschüttelt, die vereinigten Methylenchloridphasen über Natriumsulfat getrocknet, eingedampft und der ölige Rückstand unter Zugabe von Petroläther/Isopropanol zur Kristallisation gebracht.

Man erhält so 4,3 g = 80 % der Theorie cis-2-(Imidazolyl-1-methyl)-2-(2,4-dichlorphenyl)-1,3-dioxolan-4-yl-[4-methylthio-(morpholinommethylen-anilin)] in Form eines weißen, kristalinen Pulvers von Fp. 132°C.

Das als Ausgangsstoff verwendete cis-2-(Imidazolyl-1-methyl)-2-(2,4-dichlorphenyl)-1,3-dioxolan-4-yl-(4-methylthioanilin) wird nach dem in Europ.Pat.52 905 beschriebenen Verfahren oder nach dem auf Seite 43 beschriebenen Verfahren dargestellt.

cis-2-(Imidazolyl-1-methyl)-2-(2,4-dichlorphenyl)-1,3-dioxolan-4-yl-(4-methylthio-anilin)

46,6 g (0,1 Mol) cis-2-(Imidazolyl-1-methyl)-2-(2,4-dichlorphenyl)-1,3-dioxolan-4-yl-(4-methylthio-nitrobenzol) werden in 500 ml Äthanol gelöst, 12,0 g (0,11 Mol) Calciumchlorid gelöst in 50 ml Wasser und 100 ml Äthanol zugegeben und unter gutem Rühren bei 25°C (leicht exotherme Reaktion!) 32,5 g (0,5 Mol) Zink-Staub portionsweise eingetragen. Ist alles eingetragen, wird die Reduktionsmischung 2 Stunden unter Rückfluß gerührt. Nach dem Abkühlen, wird vom unverbrauchten Zink-Staub abfiltriert und die Lösung am Rotationsverdampfer unter vermindertem Druck eingedampft. Der Rückstand wird aus Toluol umkristallisiert. Man erhält so 31 g = 71 % der Theorie cis-2-(Imidazolyl-1-methyl)-2-(2,4-dichlorphenyl)-1,3-dioxolan-4-yl-(4-methylthio-anilin) in Form eines zähen Öles.

Die Substanz ist identisch mit der nach Europ.Pat. 52 905 erhaltenen Verbindung und rein genug für weitere Umsetzungen.

Die als Ausgangsstoff verwendete Nitroverbindung wird wie folgt dargestellt:

cis-2-(Imidazolyl-1-methyl)-2-(2,4-dichlorphenyl)-1,3-dioxolan-4-yl-(4-methylthio-nitrobenzol)

47,9 g (0,1 Mol) cis-2-(Brommethyl)-2-(2,4-dichlorphenyl)-1,3-dioxolan-4-yl-(4-methylthio-nitrobenzol) werden zusammen mit 20,4 g (0,3 Mol) Imidazol in 500 ml Dimethylformamid 3 Tage unter Rückfluß erhitzt. Die Reaktionsmischung wird am Rotationsverdampfer und vermindertem Druck eingedampft, der Imidazol-Überschuß durch Anrühren mit Wasser extrahiert, der Rückstand in Methylenchlorid gelöst und unter Zusatz von Methanol (20 : 1) über eine Säule beladen mit Kieselgel chromatographiert.

Nach Eindampfen der chromatographierten Lösung und Umkristallisation des Rückstandes aus wenig Äthanol erhält man cis-2-(Imidazolyl-1-methyl)-2-(2,4-dichlorphenyl)-1,3-dioxolan-4-yl-(4-methylthio-nitrobenzol).

Die Substanz ist identisch mit der durch Umsetzung von cis-2-(Imidazolyl-1-methyl)-2-(2,4-dichlorphenyl)-1,3-dioxolan-4-yl-methanol-mesylat (nach DOS 2.804.096) mit 4-Nitrothiophenol-Natrium-Salz gewonnenen Verbindung.

Das als Ausgangsstoff verwendete cis-2-Brommethyl-2-(2,4-dichlorphenyl)-1,3-dioxolan-4-yl-(4-methylthio-nitrobenzol) wird wie unter Verfahren C beschrieben dargestellt.

Das nach Beispiel 3.1 hergestellte cis-2-(Imidazolyl-1-methyl)-2-(2,4-dichlorphenyl)-1,3-dioxolan-4-yl-(4-methylthiomorpholinomethylen-anilin) wird auch wir folgt erhalten:

Verfahren A b)

Durch Umsetzung von cis-2-(Imidazolyl-1-methyl)-2-(2,4-dichlorphenyl)-1,3-dioxolan-4-yl-(4-methylthio-anilin) mit N-Formyl-morpholin-diäthylacetal in Pyridin bei 50°C.

Verfahren A c)

Diese Verfahrensvariante ist zur Darstellung von cis-2-(Azolyl-1-methyl)-2-(subst.-phenyl)-1,3-dioxolan-4-yl-(4-methylthio-(monoalkylamino-methylen-anilinen) geeignet, wie sie in den Beispielen genannt werden.

Man erhält diese Verbindungen durch Umsetzung von cis-2-(Imidazolyl-1-methyl)-2-(2,4-dichlorphenyl)-1,3-dioxolan-4-yl-(4-methylthio-anilin) mit entsprechenden Imidoäthern (Formel V) in Eisessig oder Dimethylformamid bei 120°C.

Verfahren A d)

Durch Umsetzung von cis-2-(Imidazolyl-1-methyl)-2-(2,4-dichlorphenyl)-1,3-dioxolan-4-yl-(4-methylthio-anilin) mit Orthoameisensäuretrimethylester ohne Verdünnungsmittel in Gegenwart von etwas Essigsäureanhydrid bei 100°C und anschließender Umsetzung des so gewonnenen Imidoäthers mit Morpholin ohne Verdünnungsmittel bei 100°C.

Verfahren A e)

Durch Umsetzung von cis-2-(Imidazolyl-1-methyl)-2-(2,4-dichlorphenyl)-1,3-dioxolan-4-yl-(4-methylthio-anilin) mit wasserfreiem Trichloracetaldehyd (Chloral) in Polyphosphorsäure in Gegenwart von Phosphorpentoxid bei 70°C und anschließender Umsetzung der so erhaltenen Trichloräthyliden-Verbindung mit Morpholin ohne Verdünnungsmittel bei 100°C.

Die Verfahren A d) und A e) werden vorteilhaft bei der Darstelllung der Piperazino-Verbindungen, wie sie im Beispiel 3.7 genannt wird, angewendet.

Verfahren A f)

Diese Verfahrensvariante ist zur Darstellung von cyclischen Amidinen geeignet, wie sie im Beispiel 3.9 genannt wird.

Man erhält diese Verbindungen durch Umsetzung von Imidchloriden (Formel XI) mit Aminen (Formel VIII) in Äthylenglycoldimethyläther bei 85°C.

Die Imidchloride werden durch Umsetzung der entsprechenden Amide (Formel X) mit Phosphorpentachlorid in Toluol bei 80°C erhalten.

Verfahren A g)

Die Verfahrensvariante ist zur Darstellung von Amidinen geeignet wie sie in den Beispielen 3.1 und folgende 1.14 genannt werden.

Man erhält diese Verbindungen durch Umsetzung von Imidchloriden der Formel XIII mit primären Aminen der Formel XIV in Äthylenglycoldimethyläther bei 85°C.

Die Imidchloride werden durch Umsetzung der entsprechenden Amide der Formel XII mit Phosphorpentachlorid in Toluol bei 80°C erhalten.

Verfahren B a)

Durch Umsetzung von 5,14 g (0,01 Mol) cis-2-(Imidazolyl-1-methyl)-2-(2,4-dichlorphenyl)-1,3-dioxolan-4-methanol-(4-nitrobenzolsulfonsäureester) (Formel XV) mit 2,22 g (0,01 Mol) 4-Morpholinomethylenimino-thiophenol (Formel XVI) in Gegenwart von 1,38 g (0,01 Mol) Kaliumcarbonat in Dimethylformamid bei 40°C erhält man die Verbindung nach Beispiel 1.1 mit indentischem Fp. 132°C. in 60 %iger Ausbeute.

Den als Ausgangsstoff verwendeten 4-Nitrobenzolsulfonsäureester erhält man durch Umsetzung von molaren Mengen cis-2-(Imidazolyl-1-methyl)-2-(2,4-dichlorphenyl)-1,3-dioxolan-4-methanol mit 4-Nitrobenzolsulfonsäurechlorid in Gegenwart von Triäthylamin.

4-Morpholinomethyleniminothio-phenol erhält man durch saure Spaltung des entsprechenden Thiophenol-S-benzyläthers. Dieser wird dargestellt durch Umsetzung von 4-Benzylthioanilin mit N-Formyl-morpholin in Gegenwart von Phosphoroxichlorid bei 40°C.

Verfahren B b)

Durch Umsetzung von 5,43 g (0,01 Mol) cis-2-Brommethyl-2-(2,4-dichlorphenyl)-1,3-dioxolan-4-methanol-(4-nitrobenzolsulfonsäureester) (Formel XVII) mit 2,22 g (0,01 Mol) 4-Morpholinomethyleniminothio-phenol (Formel XVI) in Gegenwart von 1,38 g (0,01 Mol) Kalciumcarbonat in Dimethylformamid bei 40°C erhält man cis-2-Brommethyl-2-(2,4-dichlorphenyl)-1,3-dioxolan-4-yl-/4-methoxy-(morpholinomethylen-anilin)7 in 72 %iger Ausbeute, welches mit überschüssigem Imidazol in Dimethylformamid bei 100°C (24 Stunden Rückfluß) zur Verbindung nach Beispiel 3.1 mit indentischem Fp. 132°C mit 48 %iger Ausbeute führt.

Den als Ausgangsstoff verwendeten 4-Nitrobenzolsulfonsäureester erhält man durch Umsetzung von molaren Mengen cis-2-Brommethyl-2-(2,4-dichlorphenyl)-1,3-dioxolan-4-methanol mit 4-Nitrobenzolsulfonsäurechlorid in Gegenwart von Pyridin.

Verfahren C)

Beispiel 3.2

cis-2-(Imidazolyl-1-methyl)-2-(2,4-dichlorphenyl)-1,3-dioxolan-4-yl-/4-methylthio-(morpholinomethylen-anilin)7

5,46 g (0,01 Mol cis-2-Brommethyl-2-(2,4-dichlorphenyl)-1,3-dioxolan-4-yl-/4-methylthio-(morpholinomethylen-anilin)7 werden zusammen mit 2,0 g (0,03 Mol) Imidazol in 50 ml Dimethylformamid 3 Tage unter Rückfluß erhitzt und wie unter Verfahren A) a) beschrieben aufgearbeitet. Die so erhaltene Titelverbindung ist identisch (Fp. 132°C) mit der nach Verfahren A) a) erhaltenen.

Das als Ausgangsstoff verwendete cis-2-Brommethyl-2-(2,4-dichlorphenyl)-1,3-dioxolan-4-yl-/4-methylthio-(morpholinomethylen-anilin)7 wird dargestellt durch Umsetzung von

4,5 g (0,01 Mol) cis-2-Brommethyl-2-(2,4-dichlorphenyl)-1,3-dioxolan- 4-yl-(4-methylthio-anilin) und dem Vilsmeier-Komplex aus 29,0 g N-Formyl-morpholin (Mol.-Gew. 115) und 1,85 g Phosphoroxichlorid (Mol.-Gew. 154), 2 Stunden 25°C wie unter Verfahren A) a) beschrieben.

Das hierbei als Ausgangsstoff verwendete cis-2-Brommethyl-2-(2,4-dichlorphenyl)-1,3-dioxolan-4-yl-(4-methoxy-anilin) wird dargestellt durch Reduktion von 4,79 g (0,01 Mol) cis-2-Brommethyl-2-(2,4-dichlorphenyl)-1,3-dioxolan-4-yl-(4-methylthio-nitrobenzol) mittels 3,25 g (0,05 Mol) Zink-Staub und 1,2 g (0,011 Mol) Calciumchlorid in siedendem Äthanol.

Das als Ausgangsstoff verwendete cis-2-(Brommethyl-2-(2,4-dichlorphenyl)-1,3-dioxolan-4-yl-(4-methylthio-nitrobenzol) wird wie folgt dargestellt:

26,8 (0,1 Mol) α-Brom-2,4-dichloracetophenon,
22,9 (0,1 Mol) α-Glycerin-4-nitrophenylthioäther und
1 g 4-Toluolsulfonsäure (als Kondensationskatalysator) werden in 50 ml n-Butanol und 150 ml Toluol gelöst und 12 Stunden unter Rückfluß am Wasserabscheider erhitzt. Sodann wird die Reaktionsmischung am Rotationsverdampfer im Vakuum eingedampft, der Rückstand in Methylenchlorid aufgenommen, zweimal mit 2n-Sodalösung ausgeschüttelt, die Methylenchloridphase abgetrennt, über Natriumsulfat getrocknet, eingedampft und der Rückstand aus Toluol umkristallisiert.

Man erhält so cis-2-Brommethyl-2-(2,4-dichlorphenyl)-1,3-dioxolan-4-yl-(4-methylthio-nitrobenzol).

Der als Ausgangsstoff verwendete α-Glycerin-4-nitrophenylthioäther wird durch Umsetzung von molaren Mengen 3-Chlor-1,2-dihydroxypropan und 4-Nitrothiophenol-Natriumsalz in abs. Äthanol 24 Stunden Rückfluß hergestellt. Die Verbindung ist ein gelbes Öl.

Nach den in Beispiel 3.1 beschriebenen Verfahren werden erhalten:

3.3) Cis-2-(Imidazolyl-1-methyl)-2-(2,4-dichlorphenyl)-1,3-dioxolan-4-yl-/4-methylthio-(N-dimethylamino-methylenanilin)7 Fp. 128$^{o}$C) aus Cis-2-(Imidazolyl-1-methyl)-2-(2,4-dichlorphenyl)-1,3-dioxolan-4-yl-(4-methylthio-anilin) = E und N-Dimethylformamid.

3.4) Cis-2-(Imidazolyl-1-methyl)-2-(2,4-dichlorphenyl)-1,3-dioxolan-4-yl-/4-methylthio-(N-diäthylaminomethylen-anilin)7 aus E und N-Diäthylformamid.

3.5) Cis-2-(Imidazolyl-1-methyl)-2-(2,4-dichlorphenyl)-1,3-dioxolan-4-yl-/4-methylthio-(pyrrolidinomethylen-anilin)7 aus E und N-Formyl-pyrrolidin.

3.6) Cis-2-(Imidazolyl-1-methyl)-2-(2,4-dichlorphenyl)-1,3-dioxolan-4-yl-/4-methylthio-(piperidinomethylen-anilin)7 aus E und N-Formylpiperidin.

3.7) Cis-2-(Imidazolyl-1-methyl)-2-(2,4-dichlorphenyl)-1,3-dioxolan-4-yl-/4-methylthio-(N-methylpiperazino methylen-anilin)7 aus E und N-Formyl-N'-methyl-piperazin.

3.8) Cis-2-(Imidazolyl-1-methyl)-2-(2,4-dichlorphenyl)-1,3-dioxolan-4-yl-/4-methylthio-(N-dimethylamino-1-äthylen-anilin)7 aus E und N-Dimethyl-acetamid.

3.9) Cis-2-(Imidazolyl-1-methyl)-2-(2,4-dichlorphenyl)-1,3-dioxolan-4-yl-/4-methylthio-(N-methylpyrrolidon-2-imino-benzol)7 aus E und N-Methyl-pyrrolidon.

3.10) Cis-2-(Imidazolyl-1-methyl)-2-(2,4-dichlorphenyl)-1,3-dioxolan-4-yl-/2-methyl-4-methylthio-(N-dimethylamino-methylenanilin)7
aus Cis-2-(Imidazolyl-1-methyl)-2-(2,4-dichlorphenyl)-1,3-dioxolan-4-yl-(2-methyl-4-methylthio-anilin) und N-Dimethylformamid.

3.11) Cis-2-(Imidazolyl-1-methyl)-2-(2,4-dichlorphenyl)-1,3-dioxolan-4-yl-/2-methyl-4-methoxy-(morpholinomethylen-anilin)7 Öl,Fp. 100°C
aus Cis-2-(Imidazolyl-1-methyl)-2-(2,4-dichlorphenyl)-1,3-dioxolan-4-yl-(2-methyl-4-methylthio-anilin) und N-Formylmorpholin.

3.12) Cis-2-(Imidazolyl-1-methyl)-2-(2,4-dichlorphenyl)-1,3-dioxolan-4-yl-/3-methyl-4-methylthio-(N-dimethylamino-methylenanilin)7
aus Cis-2-(Imidazolyl-1-methyl)-2-(2,4-dichlorphenyl)-1,3-dioxolan-4-yl-(3-methyl-4-methylthio-anilin) und N-Dimethylformamid.

3.13) Cis-2-(Imidazolyl-1-methyl)-2-(2,4-dichlorphenyl)-1,3-dioxolan-4-yl-/3-methyl-4-methylthio-(morpholinomethylen-anilin)7
aus Cis-2-(Imidazolyl-1-methyl)-2-(2,4-dichlorphenyl)-1,3-dioxolan-4-yl-(3-methyl-4-methylthio-anilin) und N-Formylmorpholin.

3.14) Cis-2-(Imidazolyl-1-methyl)-2-(2,4-dichlorphenyl)-1,3-dioxolan-4-yl-/2-chlor-4-methylthio-(N-dimethylamino methylen-anilin)7
aus Cis-2-(Imidazolyl-1-methyl)-2-(2,4-dichlorphenyl)-1,3-dioxolan-4-yl-(2-chlor-4-methylthio-anilin) und N-Dimethylformamid.

3.15.) Cis-2-(Imidazolyl-1-methyl)-2-(2,4-dichlorphenyl)-1,3-dioxolan-4-yl-/2-chlor-4-methylthio-(morpholinomethylenanilin)7
aus Cis-2-(Imidazolyl-1-methyl)-2-(2,4-dichlorphenyl)-1,3-dioxolan-4-yl-(2-chlor-4-methylthio-anilin) und N-Formylmorpholin.

Beispiel 4

Cis-2-(Imidazolyl-1-methyl)-2-(2,4-dichlorphenyl)-1,3-dioxolan-4-yl-/4-methylsulfinyl-(morpholinomethylen-anilin)7

5,33 g (0,01 Mol) Cis-2-(Imidazolyl-1-methyl)-2-(2,4-dichlorphenyl)-1,3-dioxolan-4-yl-/4-methylthio-(morpholinomethylen-anilin)7 werden in 50 ml Chloroform gelöst und unter Rühren zu 1,73 g (0,01 Mol) 3-Chlorperbenzoesäure gelöst in 20 ml Chloroform bei 25°C zugetropft. Das Reaktionsgemisch wird 1 Stunde bei 25°C gerührt, die Reaktionslösung mit verdünnter Natriumcarbonat-Lösung ausgeschüttelt, die Chloroformphase abgetrennt, über Natriumsulfat getrocknet, eingedampft und der Rückstand aus Alkohol umkristallisiert.

Man erhält so 3,5 g (63 % der Theorie) Cis-2-(Imidazolyl-1-methyl)-2-(2,4-dichlorphenyl)-1,3-dioxolan-4-yl-/4-methylsulfinyl-(morpholinomethylen-anilin)7 in Form von weißen Kristallen vom Fp. 145°C.

Beispiel 5

Cis-2-(Imidazolyl-1-methyl)-2-(2,4-dichlorphenyl)-1,3-dioxolan-4-yl-/4-methylsulfonyl-(morpholinomethylen-anilin)7

5,33 g (0,01 Mol) Cis-2-(Imidazolyl-1-methyl)-2-(2,4-dichlorphenyl)-1,3-dioxolan-4-yl-/4-methylthio-(morpholinomethylen-anilin)7 werden in 50 ml Eisessig gelöst und unter

Rühren 2,0 g (0,02 Mol) 35%ig wässriges Wasserstoffsuperoxid gelöst in 20 ml Eisessig bei 25°C zugetropft. Die Reaktionsmischung wird 12 Stunden bei 25°C gerührt und wie unter Beispiel 2 angegeben aufgearbeitet.

Man erhält so 4,3 g (76 % der Theorie) Cis-2-(Imidazolyl)-1-methyl)-2-(2,4-dichlorphenyl)-1,3-dioxolan-4-yl-[4-methylsulfonyl-(morpholinomethylen-anilin)] in Form von weißen Kristallen vom Fp.173°C.

Patentansprüche

1. 1-(1,3-Dioxolan-2-yl-methyl)-1H-imidazole und -1H-1,2,4-triazole der Formel I

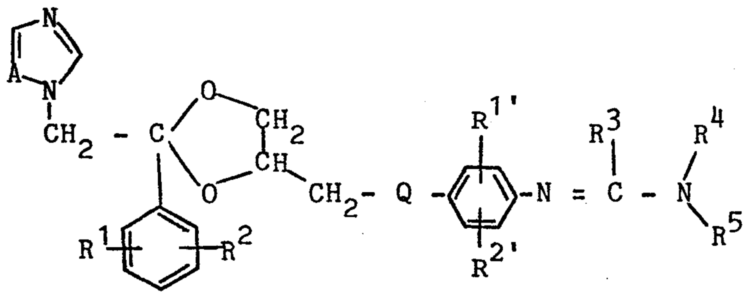 (I)

und ihre Salze mit physiologisch verträglichen Säuren, worin A ein Stickstoffatom oder eine Methingruppe, Q Sauerstoff, Schwefel, eine SO-Gruppe (Sulfoxid) oder eine $SO_2$-Gruppe (Sulfon), $R^1$ und $R^2$, $R^{1'}$ und $R^{2'}$ gleich oder verschieden sein können und Wasserstoff, Halogen, $C_1$-$C_4$-Alkyl, $C_1$-$C_4$-Alkoxy, oder Trifluormethyl, $R^3$, $R^4$, $R^5$ gleich oder verschieden sein können und Wasserstoff, geradkettiges oder verzeigtes Alkyl mit 1 bis 4 Kohlenstoffatomen bedeuten, oder worin $R^3$ und $R^4$ als Alkylenkette mit drei bis fünf Kohlenstoffatomen zusammen mit dem Stickstoff- und Kohlenstoffatom der Amidinogruppe Bestandteil eines Pyrrolidin-, Piperidin- oder Hexamethylenimin-Ringes sind, oder worin $R^4$ und $R^5$ zusammen eine Alkylenkette mit vier bis sechs Kohlenstoffatomen, die mit dem Stickstoffatom einen fünf- bis siebengliedrigen heterocyclischen Ring bilden, der ein weiteres Heteroatom aus der Gruppe Stickstoff, Sauerstoff oder Schwefel enthalten kann, insbesondere den Morpholinring, und seinerseits durch Alkyl mit ein bis drei Kohlenstoffatomen oder Aralkyl oder Aryl substituiert sein kann, bedeuten.

2. Verfahren zur Herstellung von 1-(1,3-Dioxolan-2-yl-methyl) 1H-imidazolen und -1H-1,2,4-triazolen der Formel I und ihrer Salze mit physiologisch verträglichen Säuren, dadurch gekennzeichnet, daß man

A) ein 4-[2-(subst.-Phenyl)-2-(1H-azol-1-yl-methyl)-1,3-dioxolan-4-yl-methoxy]-phenylamin der Formel II

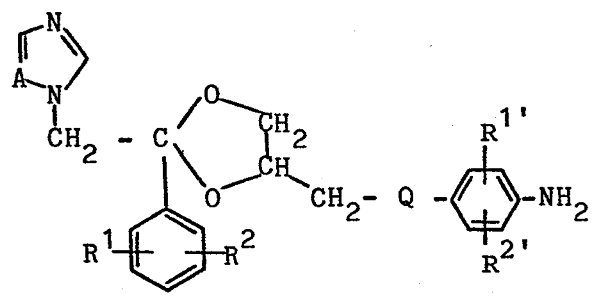

(II)

worin A,Q, $R^1$, $R^2$, $R^{1'}$ und $R^{2'}$ die zu Formel I angegebenen Bedeutungen haben,

a) mit einem Carbonsäureamid, Carbonsäurethioamid, Lactam oder Thiolactam der Formel III

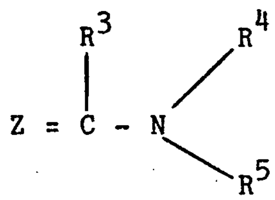

(III)

worin Z Sauerstoff oder Schwefel bedeutet und $R^3$, $R^4$ und $R^5$ die zu Formel I angegebenen Bedeutungen haben, in Gegenwart eines Kondensationsmittels umsetzt, oder

b) mit einem Acetal eines Carbonsäureamids oder eines Lactams der Formel IV

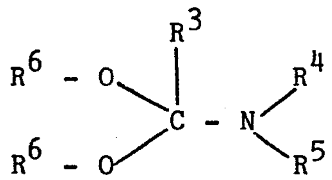

(IV)

worin $R^3$, $R^4$, $R^5$ die zu Formel I angegebenen Bedeutungen haben und $R^6$ Methyl oder Äthyl bedeutet, umsetzt, oder

c) mit einem Imidoäther oder Imidothioäther der Formel V

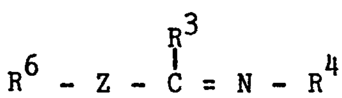

(V)

worin Z, $R^3$, $R^4$, $R^6$ die vorstehend angegebenen Bedeutungen haben, umgesetzt, oder

d) mit einem Orthoester der Formel VI

$$R^3 - C(OR^6)_3 \qquad (VI)$$

worin $R^3$ und $R^6$ die angegebenen Bedeutungen haben, in Gegenwart eines Kondensationsmittels zu einem Imidoäther der Formel VII umsetzt

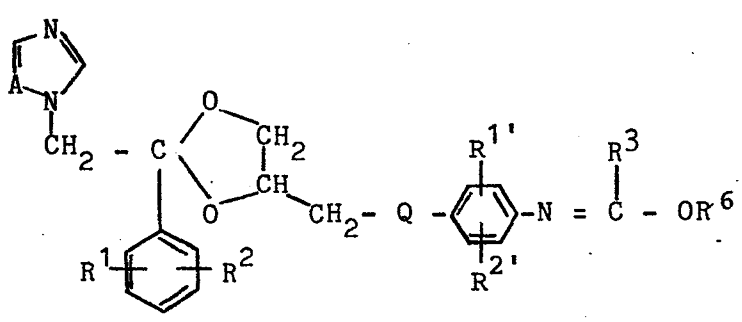

(VII)

0094052
HOE 82/F094 K

worin A,Q, $R^1$, $R^2$, $R^{1'}$, $R^{2'}$, $R^3$, und $R^6$ die angegebenen Bedeutungen haben, und anschließend die so erhaltene Verbindung VII mit einem Amin der Formel VIII

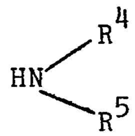

(VIII)

worin $R^4$ und $R^5$ die angegebenen Bedeutungen haben, umsetzt, oder

e) mit Trichloracetaldehyd zu einer Trichloräthyliden-Verbindung der Formel IX

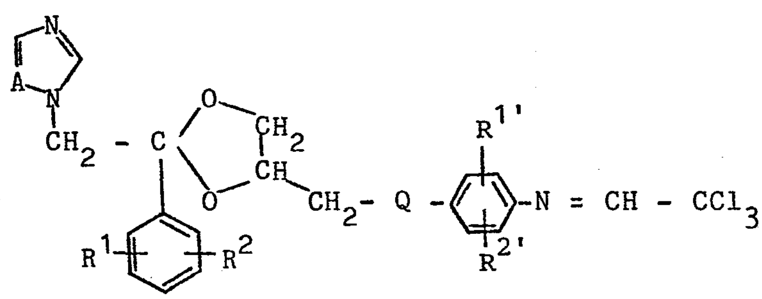

(IX)

worin A,Q, $R^1$, $R^2$, $R^{1'}$, $R^{2'}$ die angegebenen Bedeutungen haben, umsetzt und anschließend mit einem Amin der Formel VIII umsetzt, oder

f) mit einem entsprechenden Säurechlorid oder Säureanhydrid zu einem Amid der Formel X

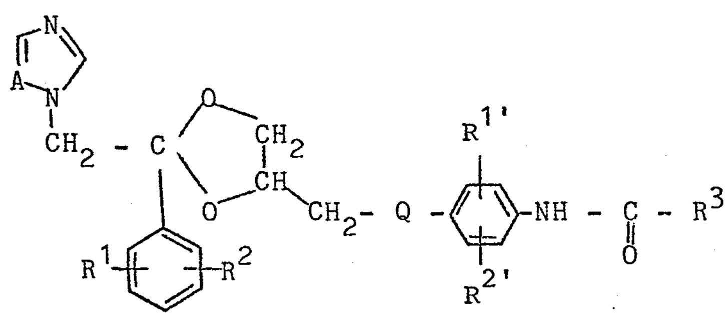

(X)

0094052

HOE 82/F 094 K

worin A,Q, $R^1$, $R^2$, $R^{1'}$, $R^{2'}$ und $R^3$, die angegebenen Bedeutungen haben umsetzt, und anschließend die Verbindung X mit einem Phosphorchlorid zu einem Imidchlorid der Formel XI

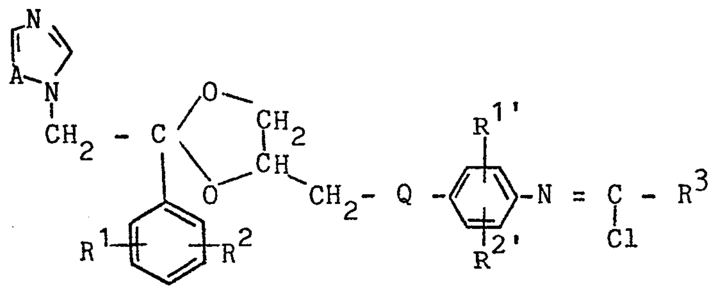

(XI)

worin A,Q, $R^1$, $R^2$,$R^{1'}$, $R^{2'}$, $R^3$ die angegebenen Bedeutungen haben, umsetzt und dieses mit einem Amin der Formel VIII umsetzt, oder

g) mit einem ω-Halogencarbonsäurechlorid oder -anhydrid zu einem Amid der Formel XII

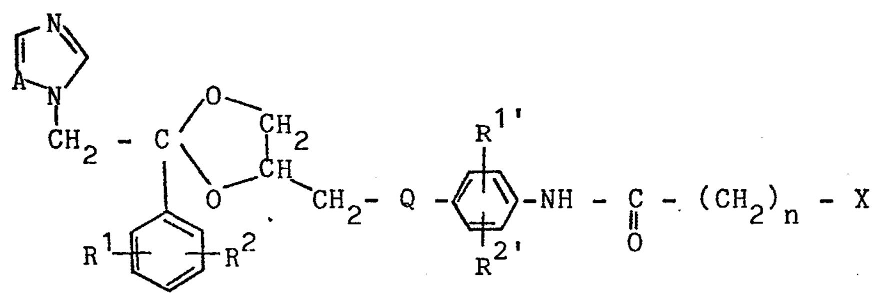

(XII)

worin A,Q, $R^1$, $R^2$, $R^{1'}$, $R^{2'}$ die angegebenen Bedeutungen haben und n die Zahl drei bis fünf und X Halogen oder Acyloxy, Alkylsulfonyloxy, oder Arylsulfonyloxy bedeutet, umsetzt und anschließend dieses mit einem Phosphorchlorid zu einem Imidchlorid der Formel XIII

0094052
HOE 82/F 094 K

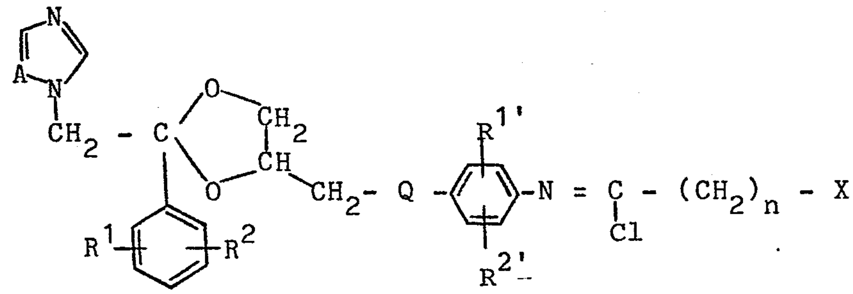

(XIII)

worin A,Q, $R^1$, $R^2$, $R^{1'}$, $R^{2'}$, n und X die angegebenen Bedeutungen haben, umsetzt und anschließend dieses mit einem Amin der Formel XIV

$H_2N - R^4$ (XIV)

worin $R^4$ die angegebene Bedeutung hat, umsetzt, oder daß man

B)a) ein 2-(subst.-Phenyl)-2-(1H-azol-1-yl-methyl)-1,3-dioxolan der Formel XV

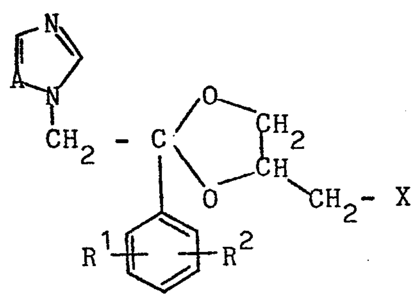

(XV)

worin A, $R^1$, $R^2$, und X die angegebenen Bedeutungen haben, mit einem 4-Amidinophenol oder mit seinem Alkalimetall- oder Ammoniumsalz der Formel XVI

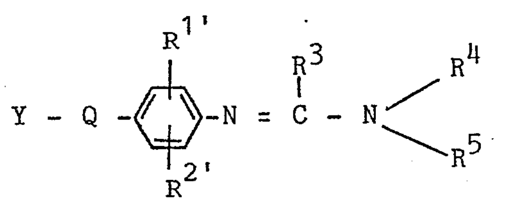

(XVI)

worin Y Wasserstoff, ein Alkalimetall, insbesondere Natrium oder Kalium, oder Ammonium bedeutet und Q, $R^{1'}$, $R^{2'}$, $R^3$, $R^4$ und $R^5$, die angegebenen Bedeutungen haben, umsetzt, oder daß man

b) ein 2-(subst.-Phenyl)-2-brommethyl-1,3-dioxolan der Formel XVII

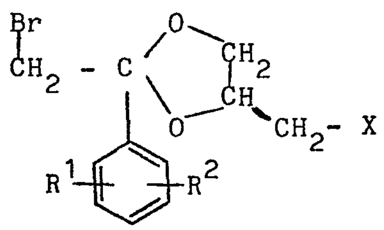

(XVII)

worin $R^1$, $R^2$ und X die angegebenen Bedeutungen haben, mit einem 4-Amidinophenol der Formel XVI zu einer Verbindung der Formel XVIII

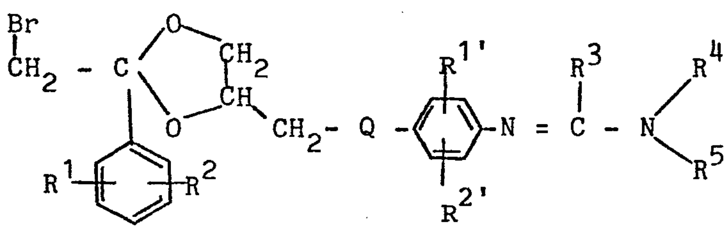

(XVIII)

worin Q, $R^1$, $R^2$, $R^{1'}$, $R^{2'}$, $R^3$, $R^4$ und $R^5$ die angegebenen Bedeutungen haben, umsetzt und diese anschließend mit einem Azol der Formel XIX

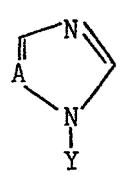

(XIX)

worin A und Y die angegebenen Bedeutungen haben, umsetzt, oder daß man

C) ein ω-Bromacetophenon der Formel XX

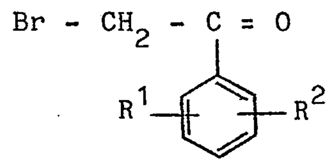

(XX)

worin $R^1$ und $R^2$ die abgegebenen Bedeutungen haben, mit α-Glycerin-4-nitrophenyläther der Formel XXI

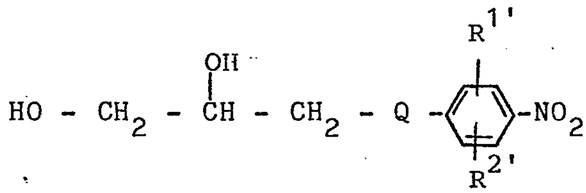

(XXI)

zu einem Ketal der Formel XXII

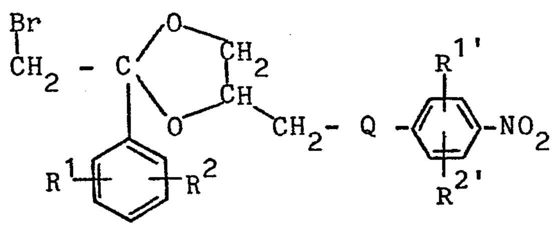

(XXII)

worin Q, $R^1$, $R^2$, $R^{1'}$ und $R^{2'}$ die angegebenen Bedeutungen haben, umsetzt, und dieses anschließend zu einer Aminoverbindung der Formel XXIII

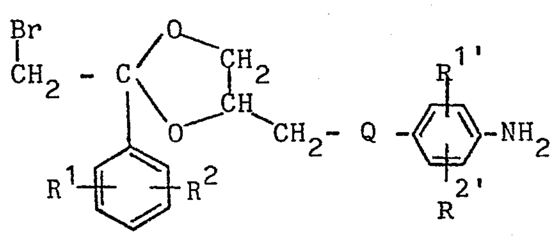

(XXIII)

worin Q, $R^1$, $R^2$, $R^{1'}$ und $R^{2'}$ die angegebenen Bedeutungen haben, reduziert und danach mit einer Verbindung der Formel III in Gegenwart eines Kondensationsmittels in eine Verbindung der Formel XVIII umwandelt und diese schließlich mit einem Azol der Formel XIX umsetzt,

und gegebenenfalls die so erhaltenen Verbindungen der Formel I durch Zugabe einer physiologisch verträglichen Säure in das Salz überführt.

3. Arzneimittel, gekennzeichnet durch einen Gehalt an einer Verbindung der Formel I in Anspruch 1.

4. Verwendung einer Verbindung der Formel I in Anspruch 1 zur Behandlung von Haut-Mykosen und System-Mykosen.

5. Verfahren zur Herstellung einer Verbindung der Formel II

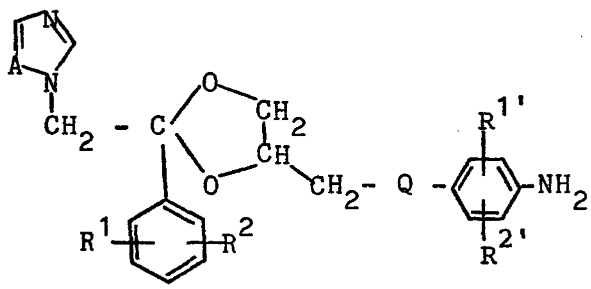

(II)

worin A ein Stickstoffatom oder eine Methingruppe bedeutet, Q Sauerstoff, Schwefel, Sulfoxid oder Sulfon bedeutet, und $R^1$, $R^2$, $R^{1'}$, $R^{2'}$ gleich oder verschieden sein können und Wasserstoff, Halogen $C_1$-$C_4$-Alkyl, $C_1$-$C_4$-Alkoxy, oder Trifluormethyl bedeuten, dadurch gekennzeichnet, daß man ein ω-Bromacetophenon der Formel XX

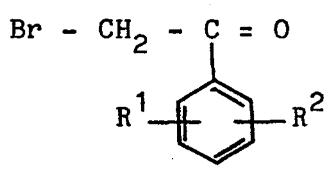

(XX)

worin $R^1$ und $R^2$ die abgegebenen Bedeutungen haben, mit ꭍ-Glycerin-4-nitrophenyläther der Formel XXI

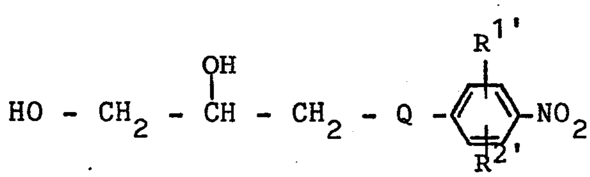

(XXI)

zu einem Ketal der Formel XXII

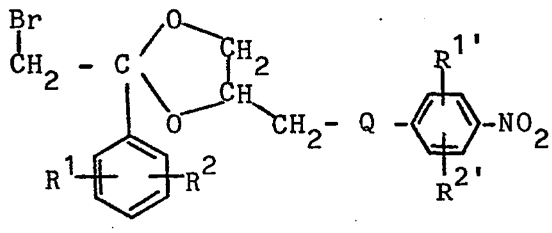

(XXII)

worin Q, $R^1$ und $R^2$, $R^{1'}$ und $R^{2'}$ die angegebenen Bedeutungen haben, umsetzt, und dieses anschließend mit einem Azol der Formel XIX

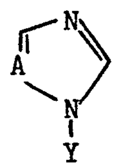

(XIX)

worin A die angegebene Bedeutung hat und Y Wasserstoff, ein Alkalimetall, insbesondere Natrium oder Kalium, oder Ammonium bedeutet, zu einer Verbindung der Formel XXIV

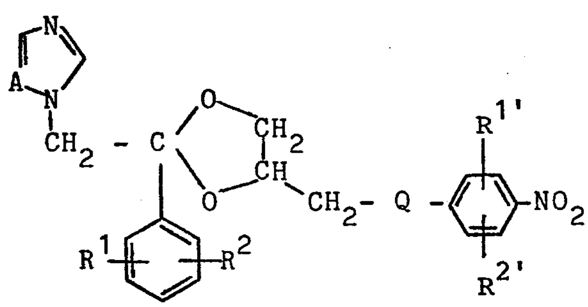

(XXIV)

worin A, Q, $R^1$ und $R^2$, $R^{1'}$ und $R^{2'}$ die angegebenen Bedeutungen haben, umsetzt, und die so erhaltene Verbindung zum entsprechenden Amin reduziert.

## Patentansprüche Österreich:

1. Verfahren zur Herstellung von 1-(1,3-Dioxolan-2-yl-methyl)-1H-imidazolen und -1H-1,2,4-triazolen der Formel I

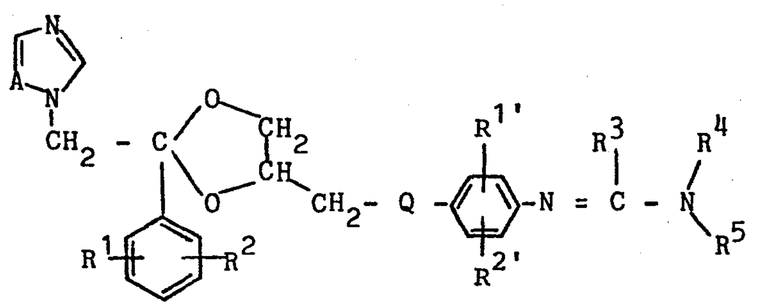

(I)

und ihrer Salze mit physiologisch verträglichen Säuren, worin A ein Stickstoffatom oder eine Methingruppe, Q Sauerstoff, Schwefel, eine SO-Gruppe (Sulfoxid) oder eine $SO_2$-Gruppe (Sulfon), $R^1$ und $R^2$, $R^{1'}$ und $R^{2'}$ gleich oder verschieden sein können und Wasserstoff, Halogen, $C_1$-$C_4$-Alkyl, $C_1$-$C_4$-Alkoxy, oder Trifluormethyl, $R^3$, $R^4$, $R^5$ gleich oder verschieden sein können und Wasserstoff, geradkettiges oder verzeigtes Alkyl mit 1 bis 4 Kohlenstoffatomen bedeuten, oder worin $R^3$ und $R^4$ als Alkylenkette mit drei bis fünf Kohlenstoffatomen zusammen mit dem Stickstoff- und Kohlenstoffatom der Amidinogruppe Bestandteil eines Pyrrolidin-, Piperidin- oder Hexamethylenimin-Ringes sind, oder worin $R^4$ und $R^5$ zusammen eine Alkylenkette mit vier bis sechs Kohlenstoffatomen, die mit dem Stickstoffatom einen fünf- bis siebengliedrigen heterocyclischen Ring bilden, der ein weiteres Heteroatom aus der Gruppe Stickstoff, Sauerstoff oder Schwefel enthalten kann, insbesondere den Morpholinring, und seinerseits durch Alkyl mit ein bis drei Kohlenstoffatomen oder Aralkyl oder Aryl substituiert sein kann, bedeuten,

dadurch gekennzeichnet, daß man

A) ein 4-[2-(subst.-Phenyl)-2-(1H-azol-1-yl-methyl)-1,3-dioxolan-4-yl-methoxy]-phenylamin der Formel II

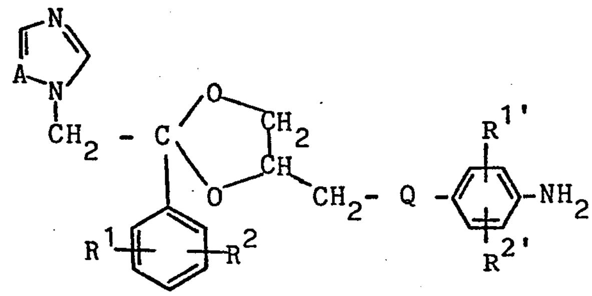

(II)

worin A,Q, $R^1$, $R^2$, $R^{1'}$ und $R^{2'}$ die zu Formel I angegebenen Bedeutungen haben,

a) mit einem Carbonsäureamid, Carbonsäurethioamid, Lactam oder Thiolactam der Formel III

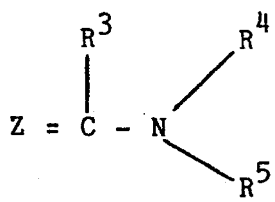

(III)

worin Z Sauerstoff oder Schwefel bedeutet und $R^3$, $R^4$ und $R^5$ die zu Formel I angegebenen Bedeutungen haben, in Gegenwart eines Kondensationsmittels umsetzt, oder

b) mit einem Acetal eines Carbonsäureamids oder eines Lactams der Formel IV

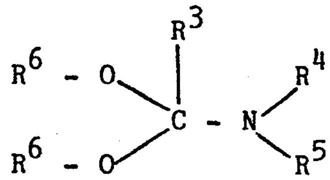

(IV)

worin $R^3$, $R^4$, $R^5$ die zu Formel I angegebenen Bedeutungen haben und $R^6$ Methyl oder Äthyl bedeutet, umsetzt, oder

c) mit einem Imidoäther oder Imidothioäther der Formel V

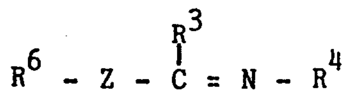

(V)

worin Z, $R^3$, $R^4$, $R^6$ die vorstehend angegebenen Bedeutungen haben, umgesetzt, oder

d) mit einem Orthoester der Formel VI

$$R^3 - C(OR^6)_3 \qquad (VI)$$

worin $R^3$ und $R^6$ die angegebenen Bedeutungen haben, in Gegenwart eines Kondensationsmittels zu einem Imidoäther der Formel VII umsetzt

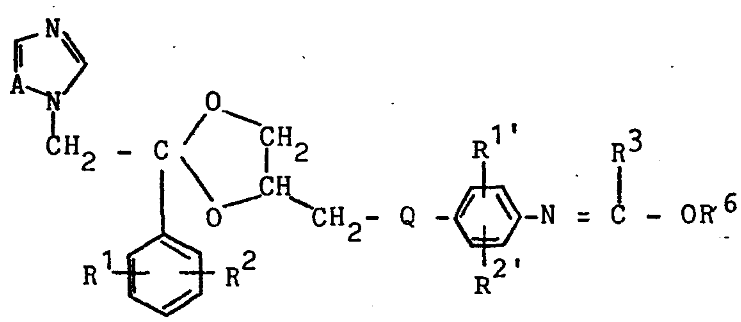

(VII)

worin A,Q, $R^1$, $R^2$, $R^{1'}$, $R^{2'}$, $R^3$, und $R^6$ die angegebenen Bedeutungen haben, und anschließend die so erhaltene Verbindung VII mit einem Amin der Formel VIII

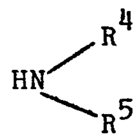

(VIII)

worin $R^4$ und $R^5$ die angegebenen Bedeutungen haben, umsetzt, oder

e) mit Trichloracetaldehyd zu einer Trichloräthyliden-Verbindung der Formel IX

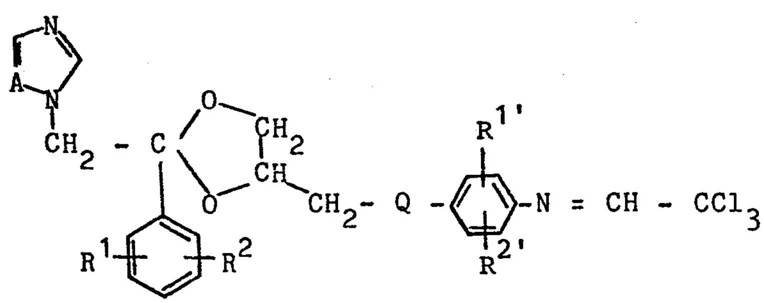

(IX)

worin A,Q, $R^1$, $R^2$, $R^{1'}$, $R^{2'}$ die angegebenen Bedeutungen haben, umsetzt und anschließend mit einem Amin der Formel VIII umsetzt, oder

f) mit einem entsprechenden Säurechlorid oder Säureanhydrid zu einem Amid der Formel X

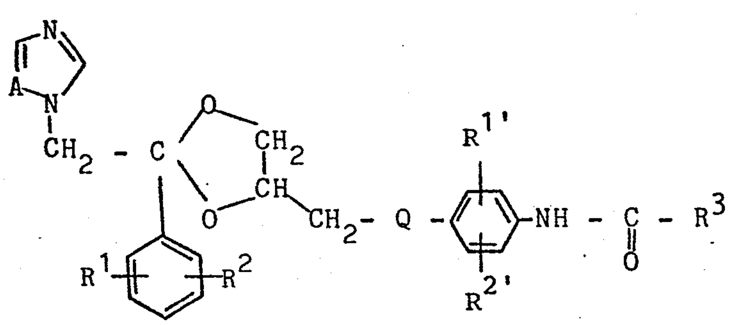

(X)

worin A,Q, $R^1$, $R^2$, $R^{1'}$, $R^{2'}$ und $R^3$, die angegebenen Bedeutungen haben umsetzt, und anschließend die Verbindung X mit einem Phosphorchlorid zu einem Imidchlorid der Formel XI

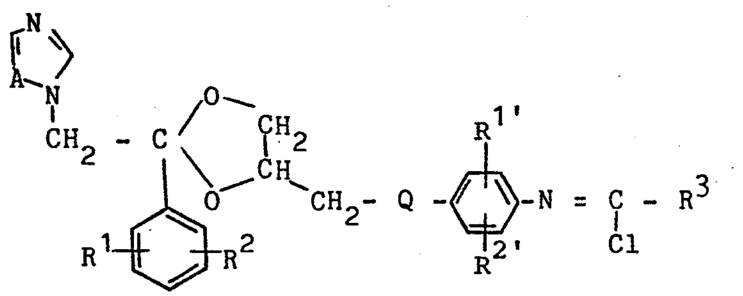

(XI)

worin A,Q, $R^1$, $R^2$,$R^{1'}$, $R^{2'}$, $R^3$ die angegebenen Bedeutungen haben,umsetzt und dieses mit einem Amin der Formel VIII umsetzt, oder

g) mit einem ω-Halogencarbonsäurechlorid oder -anhydrid zu einem Amid der Formel XII

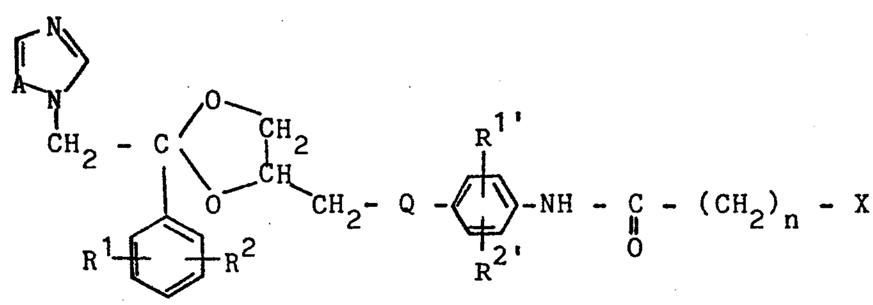

(XII)

worin A,Q, $R^1$, $R^2$, $R^{1'}$, $R^{2'}$ die angegebenen Bedeutungen haben und n die Zahl drei bis fünf und X Halogen oder Acyloxy, Alkylsulfonyloxy, oder Arylsulfonyloxy bedeutet, umsetzt und anschließend dieses mit einem Phosphorchlorid zu einem Imidchlorid der Formel XIII

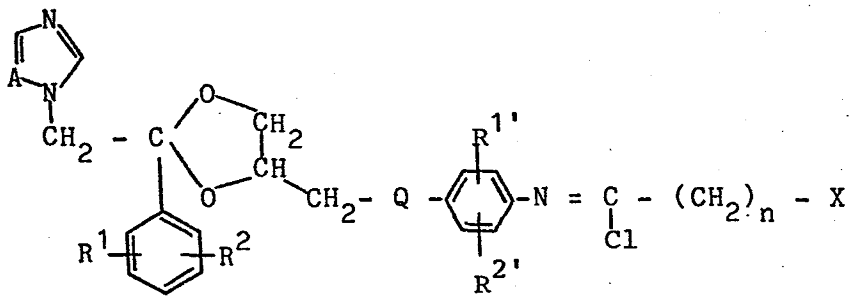

(XIII)

worin A,Q, $R^1$, $R^2$, $R^{1'}$, $R^{2'}$, n und X die angegebenen Bedeutungen haben, umsetzt und anschließend dieses mit einem Amin der Formel XIV

$$H_2N - R^4 \qquad (XIV)$$

worin $R^4$ die angegebene Bedeutung hat, umsetzt, oder daß man

B)a) ein 2-(subst.-Phenyl)-2-(1H-azol-1-yl-methyl)-1,3-dioxolan der Formel XV

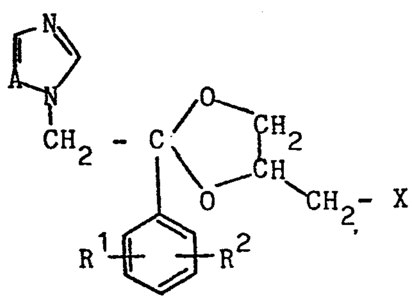

(XV)

worin A, $R^1$, $R^2$, und X die angegebenen Bedeutungen haben, mit einem 4-Amidinophenol oder mit seinem Alkalimetall- oder Ammoniumsalz der Formel XVI

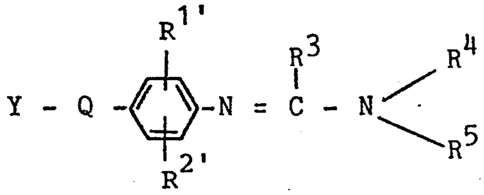

(XVI)

worin Y Wasserstoff, ein Alkalimetall, insbesondere Natrium oder Kalium, oder Ammonium bedeutet und Q, $R^{1'}$, $R^{2'}$, $R^3$, $R^4$ und $R^5$, die angegebenen Bedeutungen haben, umsetzt, oder daß man

b) ein 2-(subst.-Phenyl)-2-brommethyl-1,3-dioxolan der Formel XVII

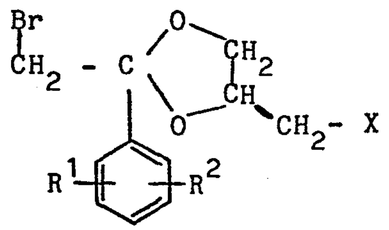

(XVII)

worin $R^1$, $R^2$ und X die angegebenen Bedeutungen haben, mit einem 4-Amidinophenol der Formel XVI zu einer Verbindung der Formel XVIII

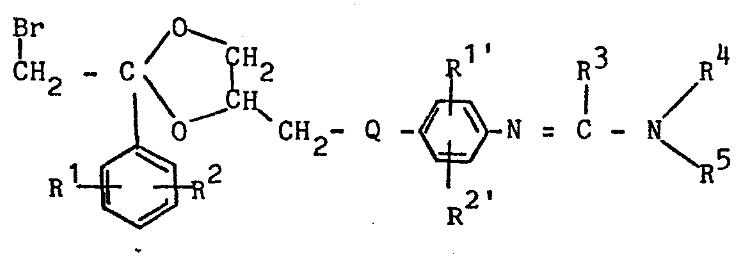

(XVIII)

worin Q, $R^1$, $R^2$, $R^{1'}$, $R^{2'}$, $R^3$, $R^4$ und $R^5$ die angegebenen Bedeutungen haben, umsetzt und diese anschließend mit einem Azol der Formel XIX

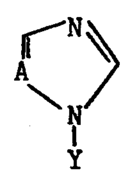

(XIX)

worin A und Y die angegebenen Bedeutungen haben, umsetzt, oder daß man

C) ein ω-Bromacetophenon der Formel XX

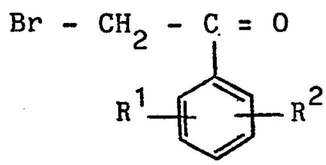

(XX)

worin $R^1$ und $R^2$ die abgegebenen Bedeutungen haben, mit α-Glycerin-4-nitrophenyläther der Formel XXI

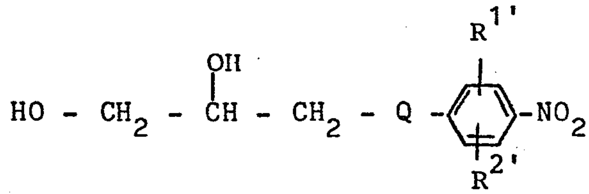

(XXI)

zu einem Ketal der Formel XXII

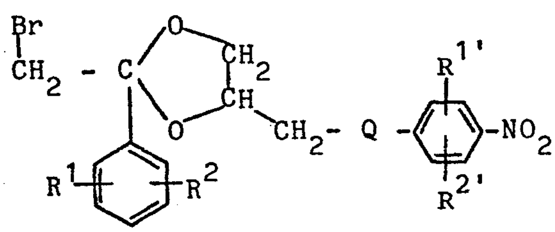

(XXII)

worin Q, $R^1$, $R^2$, $R^{1'}$ und $R^{2'}$ die angegebenen Bedeutungen haben, umsetzt, und dieses anschließend zu einer Aminoverbindung der Formel XXIII

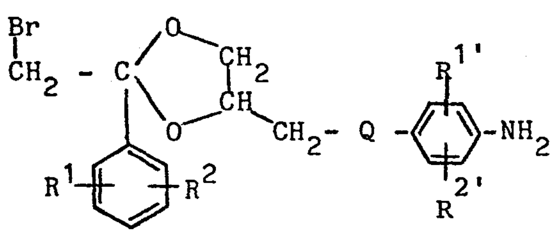

(XXIII)

worin Q, $R^1$, $R^2$, $R^{1'}$ und $R^{2'}$ die angegebenen Bedeutungen haben, reduziert und danach mit einer Verbindung der Formel III in Gegenwart eines Kondensationsmittels in eine Verbindung der Formel XVIII umwandelt und diese schließlich mit einem Azol der Formel XIX umsetzt,

und gegebenenfalls die so erhaltenen Verbindungen der Formel I durch Zugabe einer physiologisch verträglichen Säure in das Salz überführt.

2. Verfahren zur Herstellung einer Verbindung der Formel II

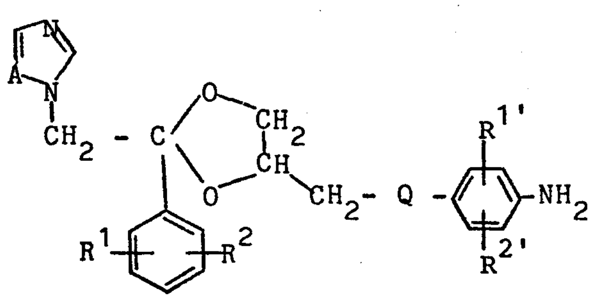

(II)

worin A ein Stickstoffatom oder eine Methingruppe bedeutet, Q Sauerstoff, Schwefel, Sulfoxid oder Sulfon bedeutet, und $R^1$, $R^2$, $R^{1'}$, $R^{2'}$ gleich oder verschieden sein können und Wasserstoff, Halogen $C_1$-$C_4$-Alkyl, $C_1$-$C_4$-Alkoxy, oder Trifluormethyl bedeuten, dadurch gekennzeichnet, daß man ein ω-Bromacetophenon der Formel XX

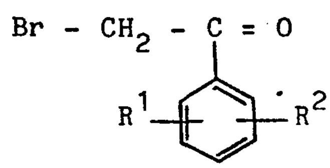

(XX)

worin $R^1$ und $R^2$ die abgegebenen Bedeutungen haben, mit α-Glycerin-4-nitrophenyläther der Formel XXI

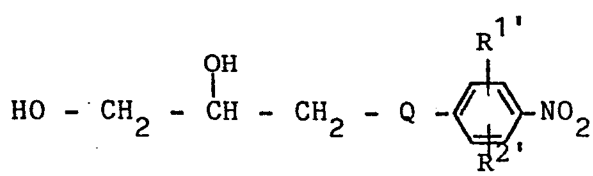

(XXI)

zu einem Ketal der Formel XXII

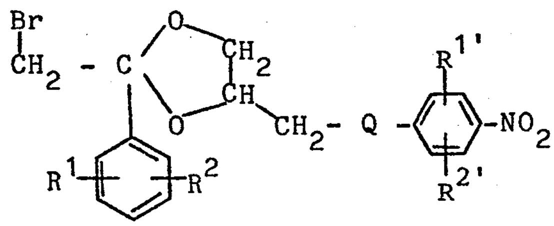

(XXII)

worin Q, $R^1$ und $R^2$, $R^{1'}$ und $R^{2'}$ die angegebenen Bedeutungen haben, umsetzt, und dieses anschließend mit einem Azol der Formel XIX

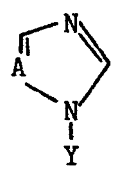

(XIX)

worin A die angegebene Bedeutung hat und Y Wasserstoff, ein Alkalimetall, insbesondere Natrium oder Kalium, oder Ammonium bedeutet, zu einer Verbindung der Formel XXIV

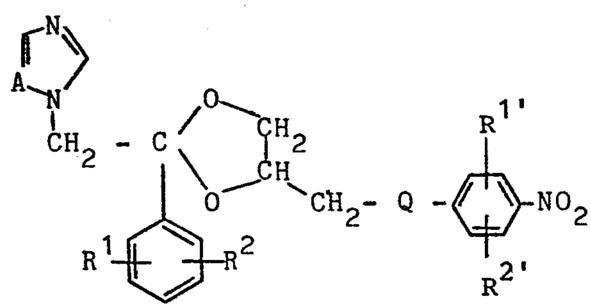

(XXIV)

worin A, Q, $R^1$ und $R^2$, $R^{1'}$ und $R^{2'}$ die angegebenen Bedeutungen haben, umsetzt, und die so erhaltene Verbindung zum entsprechenden Amin reduziert.